(19) Europäisches Patentamt
European Patent Office
Office européen des brevets

(11) **EP 4 685 809 A1**

(12) **EUROPEAN PATENT APPLICATION**

(43) Date of publication:
**28.01.2026 Bulletin 2026/05**

(21) Application number: **24306255.1**

(22) Date of filing: **25.07.2024**

(51) International Patent Classification (IPC):
**G16H 20/40** (2018.01)    **A61B 5/02** (2006.01)
**A61B 34/10** (2016.01)    **A61B 34/20** (2016.01)
**G16H 30/40** (2018.01)    **G16H 50/20** (2018.01)
**G16H 50/30** (2018.01)    **G16H 50/50** (2018.01)

(52) Cooperative Patent Classification (CPC):
**G16H 30/40; A61B 34/10; A61B 34/20;**
**G16H 20/40; G16H 50/20; G16H 50/30;**
**G16H 50/50;** A61B 2034/104; A61B 2034/105;
A61B 2034/107

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **HENDRIKS, Bernardus**
  **5656 AG Eindhoven (NL)**

• **OLIVAN BESCOS, Javier**
  **5656 AG Eindhoven (NL)**
• **FLORENT, Raoul**
  **5656 AG Eindhoven (NL)**
• **GRASS, Michael**
  **5656 AG Eindhoven (NL)**
• **VAN MOURIK, Martijn**
  **5656 AG Eindhoven (NL)**
• **ELENBAAS, Thijs**
  **5656 AG Eindhoven (NL)**

(74) Representative: **Philips Intellectual Property &**
**Standards**
**High Tech Campus 52**
**5656 AG Eindhoven (NL)**

(54) **SYSTEMS AND METHODS FOR ADJUSTMENT OF A TREATMENT PLAN USING TREATMENT PROGRESS CHARACTERISTICS FROM IMAGING DATA**

(57) A system is provided for determining a treatment plan for a medical procedure. The system includes a processor configured to generate the treatment plan for the medical procedure. The processor is also configured to obtain images acquired during performance of the medical procedure according to the treatment plan, record progression of the medical procedure based on the images, and extract treatment progress characteristics from the images. The treatment plan may be for treatment of an occlusion and the treatment progress characteristics may include at least one of: (i) occlusion proximal cap morphology, (ii) occlusion length, course, and composition, (iii) quality of a vessel distal to the occlusion, and (iv) collateral circulation. The processor is configured to determine (i) whether to adjust the treatment plan and/or (ii) a treatment selection to use to adjust the treatment plan based on the treatment progress characteristics and the progression of the medical procedure.

FIG. 2

**Description**

BACKGROUND OF THE INVENTION

Over time, a blood vessel may become occluded due to a build-up of plaque or a clot in the blood vessel. An occlusion can occur in blood vessels in various parts of the anatomy, including in coronary arteries in the heart. In some cases, an occlusion is partial and blood flow in the vessel is restricted, but not blocked. However, in more severe cases, blood flow in the vessel may be completely blocked by the occlusion. Such complete blockage of the blood vessel is referred to as a chronic total occlusion (CTO).

If a blood vessel has a CTO, a clinician may determine a treatment plan for performing an interventional treatment procedure, such as a percutaneous coronary intervention (PCI), in a catheterization laboratory (cathlab) to restore blood flow through the blood vessel. The determination of a preferred treatment plan for the treatment procedure is challenging. Before the treatment procedure, a first order treatment plan can be created using a pre-operative image, such as a coronary computed tomography (CT) angiogram (CCTA). However, such treatment plan typically requires adjustment during progression of the treatment procedure since some properties needed for accurately assessing the CTO are only detectable as a result of steps performed during the treatment procedure, and many of these steps are trial and error.

The information included in this Background section of the specification, including any references cited herein and any description or discussion thereof, is included for technical reference purposes only and is not to be regarded as subject matter by which the scope of the disclosure is to be bound.

SUMMARY OF THE INVENTION

Consequently, there is a need for an improved approach to determine whether a treatment plan requires adjustment during progression of the treatment procedure and, if so, what are the specific adjustments that need to be made to the treatment plan. To address such need to improve treatment plans, embodiments of the present disclosure create a treatment plan based on pre-operative imaging data and, during the progression of the treatment procedure, adjusts the treatment plan based on treatment progress characteristics determined from intra-operative imaging data (e.g., X-ray images). In some embodiments, the treatment progress characteristics may include one or more of: (1) proximal cap morphology of an occlusion, (2) occlusion length, course, and composition, (3) quality of the vessel distal to the occlusion, and (4) collateral circulation.

According to a representative embodiment, a system is provided for determining a treatment plan for a medical procedure. The system includes a processor in communication with memory. The processor is configured to generate the treatment plan for the medical procedure; obtain one or more images acquired during performance of the medical procedure according to the treatment plan; record progression of the medical procedure based on the one or more images; extract at least one treatment progress characteristic from the one or more images; and determine whether to adjust the treatment plan based on the at least one treatment, progress characteristic and the progression of the medical procedure.

In some embodiments, if determined to adjust the treatment plan, the processor may be further configured to determine an adjustment to a treatment selection in the treatment plan based on the at least one treatment progress characteristic and the progress of the medical procedure. In some embodiments, the processor may be configured to create the treatment plan based on assessment of one or more pre-operative coronary computed tomography angiogram (CCTA) images. In some embodiments, the one or more images acquired during the progression of the medical procedure may comprise one or more X-ray images. In some embodiments, the treatment plan may be for treatment of an occlusion of a vessel and the at least one treatment progress characteristic may include at least one of: (i) proximal cap morphology of an occlusion, (ii) occlusion length, course, and composition, (iii) quality of a vessel distal to the occlusion, and (iv) collateral circulation.

In some embodiments, the processor may be further configured to determine whether to adjust the treatment plan based on data acquired by a sensor disposed on a treatment device used to perform the medical procedure according to the treatment plan. In some embodiments, the processor may be further configured to score one or more properties of the at least one treatment progress characteristic to determine at least one of (i) whether to adjust the treatment plan and (ii) a preferred treatment selection to use to adjust the treatment plan. In some embodiments, the processor may be further configured to determine one or more properties of the at least one treatment progress characteristic based on a result of an action performed during the medical procedure, including at least one of: bending and deforming of a treatment device inserted into a vessel during progression of the medical procedure, position of the treatment device with respect to a wall of the vessel, force asserted on the treatment device in response to contact with the vessel or an occlusion in the vessel.

In some embodiments, the processor may be configured to input the treatment plan and one or more properties of the at least one treatment progress characteristic into a machine-learning model trained to predict at least one of (i) whether to adjust the treatment plan and (ii) a preferred treatment selection to use to adjust the treatment plan. In some embodiments, a second processor may be configured to train the machine-learning model based on a database of past treatment procedures and corresponding past images including the at least one treatment progress characteristic.

According to a representative embodiment, a method is provided for determining a treatment plan for a medical procedure. The method includes generating the treatment plan for the medical procedure; obtaining one or more images acquired during performance of the medical procedure according to the treatment plan; recording progression of the medical procedure based on the one or more images; extracting at least one treatment progress characteristic from the one or more images; and determining whether to adjust the treatment plan based on the at least one treatment progress characteristic and the progression of the medical procedure.

In some embodiments, if determined to adjust the treatment plan, the method may further comprise determining an adjustment to a treatment selection in the treatment plan based on the at least one treatment progress characteristic and the progress of the medical procedure. In some embodiments, the treatment plan may be for treatment of an occlusion of a vessel, and the at least one treatment progress characteristic may include at least one of: (i) proximal cap morphology of an occlusion, (ii) occlusion length, course, and composition, (iii) quality of a vessel distal to the occlusion, and (iv) collateral circulation. In some embodiments, the method may score one or more properties of the at least one treatment progress characteristic to determine at least one of (i) whether to adjust the treatment plan and (ii) a preferred treatment selection to use to adjust the treatment plan. In some embodiments, the method may input the treatment plan and one or more properties of the at least one treatment progress characteristic into a machine-learning model trained from images of past procedures to predict at least one of (i) whether to adjust the treatment plan and (ii) a preferred treatment selection to use to adjust the treatment plan.

According to a representative embodiment, a non-transitory computer-readable storage medium having stored a computer program comprising instructions for determining a treatment plan for a medical procedure. The instruction, when executed by a processor, cause the processor to generate the treatment plan for the medical procedure; obtain one or more images acquired during performance of the medical procedure according to the treatment plan; record progression of the medical procedure based on the one or more images; extract at least one treatment progress characteristic from the one or more images; and determine whether to adjust the treatment plan based on the at least one treatment progress characteristic and the progression of the medical procedure.

In some embodiments, if determined to adjust the treatment plan, the instructions, when executed by the processor, may further cause the processor to determine an adjustment to a treatment selection in the treatment plan based on the at least one treatment progress characteristic and the progress of the medical procedure. In some embodiments, the treatment plan may be for treatment of an occlusion of a vessel, and the at least one treatment progress characteristic may include at least one of: (i) proximal cap morphology of an occlusion, (ii) occlusion length, course, and composition, (iii) quality of a vessel distal to the occlusion, and (iv) collateral circulation. In some embodiments, the instructions, when executed by a processor, may further cause the processor to score one or more properties of the at least one treatment progress characteristic to determine at least one of (i) whether to adjust the treatment plan and (ii) a preferred treatment selection to use to adjust the treatment plan. In some embodiments, the instructions, when executed by a processor, may further cause the processor to input the treatment plan and one or more properties of the at least one treatment progress characteristic into a machine-learning model trained from images of past procedures to predict at least one of (i) whether to adjust the treatment plan and (ii) a preferred treatment selection to use to adjust the treatment plan.

BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

Fig. 1A illustrates a heart, including an occlusion in a target vessel of the heart, and a path for approaching the occlusion in the antegrade direction, in accordance with some embodiments of the present disclosure.

Fig. 1B illustrates the heart of Fig. 1A with the path for approaching the occlusion in the retrograde direction via a collateral vessel, in accordance with some embodiments of the present disclosure.

Fig. 2 illustrates an example occlusion in a target vessel and characteristics related to the occlusion, in accordance with some embodiments of the present disclosure.

Fig. 3 illustrates a block diagram of example treatment selections for a treatment plan to treat an occlusion, in accordance with some embodiments of the present disclosure.

Fig. 4A illustrates a block diagram of a system for creating and adjusting a treatment plan for a treatment procedure, in accordance with some embodiments of the present disclosure.

Fig. 4B illustrates a block diagram of an example treatment plan module included in some embodiments of the system in Fig. 4A.

Fig. 4C illustrates a schematic diagram of an example C-arm medical imaging device included in some embodiments of the system in Fig. 4A.

Fig. 5A illustrates a flow diagram of a computer-implemented method for creating and adjusting a treatment plan for a treatment procedure, in accordance with some embodiments of the present disclosure.

Fig. 5B illustrates a flow diagram of a computer-implemented method for scoring characteristics related to an occlusion to determine adjustment of a treatment plan, in accordance with some embodiments of the present disclosure.

Fig. 5C illustrates a flow diagram of a computer-implemented method for predicting adjustment of a treatment plan using a machine-learning model, in accordance with some embodiments of the present disclosure.

Fig. 6A illustrates a schematic diagram of training of a machine-learning model to predict adjustment of a treatment plan, in accordance with some embodiments of the present disclosure.

Fig. 6B illustrates a schematic diagram of a trained machine-learning model for predicting adjustment of a treatment plan, in accordance with some embodiments of the present disclosure.

Fig. 6C illustrates a schematic diagram of a trained machine-learning model for predicting treatment difficulty scores for adjusting a treatment plan, in accordance with some embodiments of the present disclosure.

Fig. 7A illustrates example scores for characteristics related to an occlusion, in accordance with some embodiments of the present disclosure.

Fig. 7B illustrates an example scored image of an occlusion, in accordance with some embodiments of the present disclosure.

## DETAILED DESCRIPTION OF EMBODIMENTS

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skilled in the art are within the scope of the present teachings and may be used in accordance with the representative embodiments. It is to be understood that the terminology used herein is for purposes of describing particular embodiments only, and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only, and is not intended to be limiting. As used in the specification and appended claims, the singular forms of terms "a," "an" and "the" are intended to include both singular and plural forms, unless the context clearly dictates otherwise. Additionally, the terms "comprises," and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

A treatment plan is typically used for treating a medical condition, such as an occlusion (e.g., CTO), during an interventional treatment procedure (e.g., PCI) in a procedure room (e.g., cathlab). The treatment plan includes one or more treatment selections for the treatment procedure. The treatment plan is usually created initially with first order treatment selections determined from the analysis of pre-operative imaging data (e.g., CCTA). For example, the treatment plan may initially include a first order treatment selection for the direction to approach the occlusion (e.g., antegrade) with a treatment device (e.g., guidewire) from analysis of the pre-operative imaging data.

Such treatment plan typically requires adjustment during progression of the treatment procedure since some properties needed for accurately assessing the CTO are only detectable as a result of actions (e.g., steps) performed during the treatment procedure, and many of the actions are trial and error. Embodiments of the present disclosure provide systems and methods that determine adjustments to the one or more treatment selections in the treatment plan based on analysis of intra-operative data resulting from such actions performed during the treatment procedure in relation to the occlusion. For example, the system and method of some embodiments may adjust the treatment selection of direction to approach the occlusion from antegrade to retrograde based on such analysis of the intra-operative data.

In embodiments, the intra-operative data may include intra-operative imaging data (e.g., X-ray images) which includes images of actions performed during the treatment procedure in relation to the occlusion. In some embodiments, the intra-operative data also includes measurements by a sensor (e.g., on or near the treatment device) resulting from actions performed during the treatment procedure in relation to the occlusion. In some embodiments, the intra-operative data also includes other data related to the treatment, such as properties of the treatment device, personal data of the patient (e.g., health, age, weight), etc. In some embodiments, the systems and methods determine adjustments to the treatment plan based on treatment progress characteristics related to the occlusion. The treatment progress characteristics are extracted from the intra-operative data during progression of the procedure. In some embodiments, these treatment progress characteristics include (1) the proximal cap morphology of the occlusion, (2) the occlusion length, course, and composition, (3) the quality of the vessel distal to the occlusion, and/or (4) the collateral circulation.

Based on analysis of the treatment progress characteristics, the systems and methods may determine whether adjustments need to be made to the treatment selections in the treatment plan and, if so, what are the specific adjustments that need to be made to the treatment selections. In some example embodiments, the systems and methods score properties of the treatment progress characteristics to determine whether adjustments need to be made to the treatment selections in the treatment plan and, if so, what specific adjustments need to be made to the treatment selections. In some example embodiments, the systems and methods input the treatment progress characteristics into a machine-learning model (e.g., neural network) trained to predict whether the treatment plan needs adjustment and, if so, to predict the preferred treatment selections for the treatment plan.

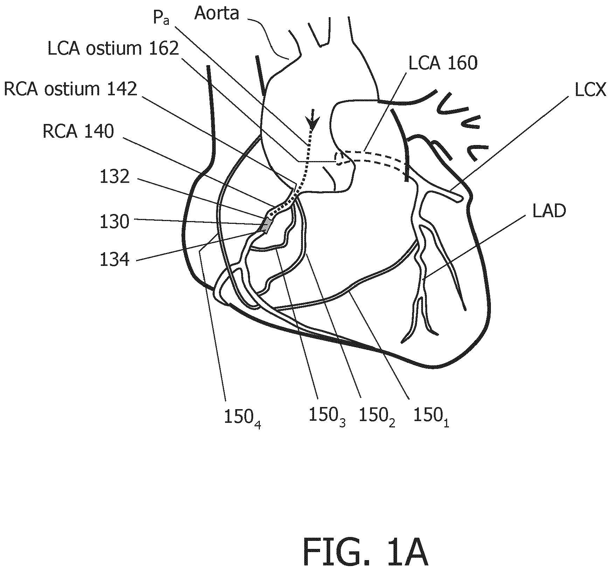

Now referring to Fig. 1A, which illustrates a heart, including an occlusion 130 in a target vessel 140 of the heart, and a path $P_a$ for approaching the occlusion 130 in the antegrade direction, in accordance with some embodiments of the present disclosure.

In the example illustrated in Fig. 1A, the occlusion 130 is located in the right coronary artery (RCA) 140 extending from the RCA ostium 142. In other embodiments, the occlusion 130 may be located in another vessel of the heart. Other arteries that supply blood to the heart are also illustrated in Fig. 1A, including the left coronary artery (LCA) 160 which extends from the LCA ostium 162 until it branches into the left circumflex artery (LCX) and the left anterior descending artery (LAD). Fig. 1A also illustrates the aorta and various collateral vessels $150_{1..4}$ that interconnect the coronary arteries and provide an alternative source of blood supply to the myocardium in cases of occlusive coronary artery disease. In the example illustrated in Fig. 1A, the collateral vessels include the septal collateral vessels $150_1$, $150_2$, the epicardial collateral vessel $150_3$, and the bypass graft vessel $150_4$.

Fig. 1A also illustrates a path $P_a$ for a treatment device (e.g., guidewire, catheter, etc.) to approach the occlusion 130, in accordance with an example embodiment of the present disclosure. In the example in Fig. 1A, the path $P_a$ is determined based on pre-operative imaging data (e.g., CCTA) and approaches the occlusion 130 in the typical antegrade direction, as indicated by the associated arrow. In the antegrade direction (i.e., the direction of conventional blood flow), the treatment device enters the occlusion 130 at or near its proximal cap 132 and exits the occlusion 130 at or near its distal cap 134 into a distal vessel.

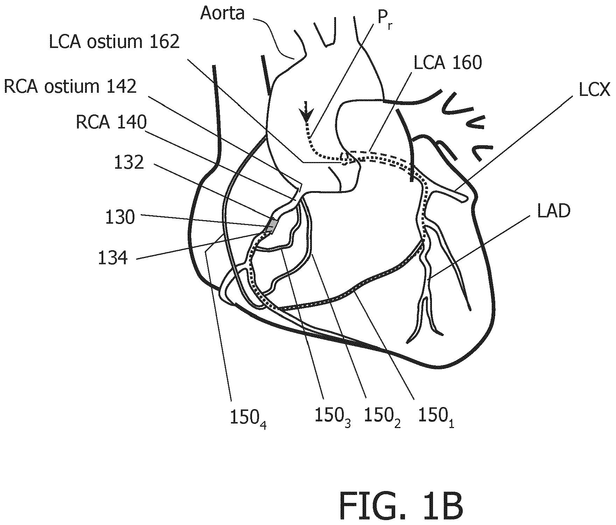

Fig. 1B illustrates the heart of Fig. 1A with the adjusted path $P_r$ for approaching the occlusion 130 in the retrograde direction, in accordance with some embodiments of the present disclosure.

The heart illustrated in Fig. 1B corresponds to the heart illustrated in Fig. 1A. A description of the common features illustrated in both Fig. 1A and in Fig. 1B is not duplicated here for the sake of brevity. In contrast to Fig. 1A, the path $P_r$ illustrated in Fig. 1B approaches the occlusion 130 in the retrograde direction, as indicated by its associated arrow. In the retrograde direction (i.e., against the direction of conventional blood flow), the treatment device approaches the occlusion via one of the collateral vessels (septal collateral vessels $150_1$), enters the occlusion at or near its distal cap 134, and exits at or near its proximal cap 132.

In some embodiments, the path $P_a$ (antegrade direction) of Fig. 1A may have been inaccurately selected because one or more characteristics related to the occlusion 130 could not be accurately assessed in the pre-operative imaging data, resulting in a treatment plan created with an improper treatment selection of the antegrade approach for treating the occlusion 130. Actions performed during the treatment procedure may result in improved information on the occlusion characteristics, which is captured in intra-operative data. For example, captured intra-operative images of bending of an inserted guidewire on contact with the proximal cap 132 of the occlusion 130 may indicate a stiff proximal cap. Based on such improved information, embodiments may determine that the antegrade approach of Fig. 1A is not suitable for the occlusion 130 due to the proximal cap stiffness and to adjust the treatment selection to a retrograde approach of Fig. 1B (or a dual antegrade-retrograde approach, not shown).

As may be appreciated from the example illustrated in Fig. 1B, while the collateral vessels $150_{1..4}$ provide alternative paths $P_r$ to the path $P_a$ illustrated in Fig. 1A for approaching the occlusion 130, such paths $P_r$ are often difficult to detect in angiographic images because the contrast agent used to generate such images tends not to accumulate close to a CTO. The collateral vessels also typically have a relative smaller lumen size, relatively greater length, and relatively higher tortuosity as compared to the target vessel 140. Thus, the collateral vessels $150_{1..4}$ are typically considered as a second choice after an antegrade approach to the occlusion has failed. A retrograde approach to an occlusion such as that provided via the collateral vessels $150_{1..4}$, also has a higher risk of complications as compared to an antegrade approach. Consequently, to proceed with the retrograde approach, it is important to analyze characteristics of the occlusion 130 in intra-operative data, including with respect to actions performed during the treatment procedure, to select the optimal collateral vessel for approaching the occlusion 130 in the retrograde direction.

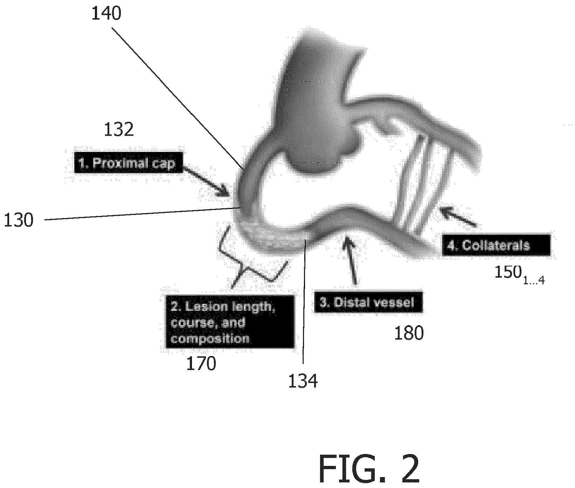

Fig. 2 illustrates an example of occlusion 130 in a target vessel 140 and characteristics related to the occlusion 130, in accordance with some embodiments of the present disclosure.

The characteristics shown in Fig. 2 include the morphology of the proximal cap 132 of the occlusion 130. Some embodiments may analyze properties related to the morphology of the proximal cap 132, such as the proximal cap location, the proximal cap shape, stiffness of the proximal cap, side-branches or bifurcations near the proximal cap 132, etc., to determine a suitable treatment selection for treating the occlusion. In some embodiments, the analysis of the proximal cap shape includes the determination of whether the occlusion is tapered shaped (e.g., funnel shaped, concave, convex), or blunt shaped (e.g., flat) which is more difficult to enter with the treatment device. The initial treatment plan may be created from pre-operative imaging data (e.g., CCTA) which provides limited visible and information of such proximal cap properties. From such limited information, the treatment plan may be created with the typical treatment selection of an antegrade approach.

Intra-operative data capturing actions performed during the treatment procedure may provide for improved analysis of the proximal cap properties. For example, during the treatment procedure, a physician may insert a guidewire into the target vessel 140 and direct the guidewire to approach and make contact with the proximal cap 132 in the target vessel 140. During the approach and contact, the guidewire may bend and deform in a particular manner due to the stiffness or shape of the occlusion 130, which may be captured in intra-operative imaging data. Also, the guidewire tip may enter the proximal cap 132 at a particular location with respect to the target vessel wall due to the shape of the occlusion 130, which may be captured in the intra-operative imaging data. In example embodiments, systems and methods may use imaging processing techniques to analyze the bending and deforming of the guidewire, the insertion location, and such indicated in the intra-operative imaging data and determine improved estimations of stiffness, shape, and/or other properties of the proximal cap morphology.

In another example, a sensor position on or near the guidewire may measure intra-operative data related to the proximal cap 132, such as the force of the guidewire when the guidewire contacts the proximal cap 132. The system may alternatively or additionally analyze the force measured by the sensor to determine an improved estimation of the stiffness, shape, and/or other properties of the proximal cap morphology. Further, in some embodiments, the intra-operative imaging data (e.g., X-ray images) provides improved visibility of the proximal cap 132 in comparison to the pre-operative imaging data, and such improved visibility enables the systems and methods to provide an improved estimation of one or more of the proximal cap 132 properties, such as shape of the proximal cap 132.

For example, based on such improved estimations from the intra-operative imaging data, the systems and methods may determine that the previously selected antegrade approach (entering the occlusion at the proximal cap 132) in the treatment plan would be of very high difficulty to perform in the treatment procedure. The systems and methods may then analyze the intra-operative imaging data to determine whether a retrograde approach (or dual antegrade-retrograde approach) would be an appropriate treatment selection to adjust the treatment plan.

The characteristics shown in Fig. 2 also include the quality of the target vessel 180 at the distal cap 134 of the occlusion 130 (also referred herein as the "distal vessel 180"). Some embodiments may determine the distal vessel quality based on analysis of properties of the distal vessel 180, such as the size of the lumen of the distal vessel 180, the presence of side-branches or bifurcation near the distal cap 134, health (e.g., damage and/or disease) of the distal vessel 180, etc., to determine a treatment selection for treating the occlusion. Some embodiments may, alternatively or additionally, analyze properties of the morphology of the distal cap 134, such as the distal cap location, the distal cap shape, stiffness of the distal cap, side-branches or bifurcations near the distal cap 132, etc. The characteristics shown in Fig. 2 also include the collateral circulation of the collateral vessels $150_{1..4}$ of the heart. Some embodiments may analyze properties related to the collateral circulation of the collateral vessels $150_{1...4}$, such as the size, tortuosity, ease of accessibility with treatment device, angle with target vessel 140, bifurcations, etc., to determine a treatment selection for treating the occlusion.

Actions performed during the treatment procedure may result in improved information on the properties of the distal cap 134, distal vessel 180, and collateral vessels $150_{1...4}$. For example, when a guidewire is inserted into a selected collateral vessel for a retrograde approach, the intra-operative imaging data may capture bending or deforming of the guidewire, angle of entry of the guidewire into the selected collateral vessel, difficulty advancing the guidewire into the selected collateral vessel, etc. In example embodiments, systems and methods may use imaging processing techniques to analyze the bending or deforming of the guidewire, angle of entry of the guidewire, difficulty advancing the guidewire, etc. indicated in the intra-operative imaging data and determine improved estimations of collateral vessel properties, such as narrowness of lumen in the collateral vessel, high tortuosity angles in the collateral vessel, etc.

In some embodiment, the intra-operative imaging data (e.g., X-ray images) may provide improved visibility of the $150_{1..4}$ in comparison to the pre-operative imaging data, and such improved visibility enables the system to provide the improved estimation of properties of the collateral circulation, such as the narrowness of lumen in the collateral vessel, high tortuosity angles in the collateral vessel, etc. Further, in some embodiments, during a retrograde approach various new contrast enhanced X-ray images of the collateral vessels may be acquired that can be analyzed to provide improved visibility of the properties of the collateral vessels.

Based on such improved estimations from the intra-operative imaging data, the systems and methods may further determine that the selected collateral vessel would be too difficult to use to approach the occlusion 130. The systems and methods may then analyze the intra-operative imaging data to select another collateral vessel for the retrograde approach in the treatment plan, or determine none of the collateral vessels are suitable to approach the occlusion 130 and adjust the treatment plan to terminate the treatment procedure (no appropriate antegrade or retrograde approach available).

Further, actions performed during the treatment procedure may result in improved information on the properties of the distal vessel and/or the distal cap 134 of the occlusion 130. For example, during the treatment procedure, a physician may insert a guidewire through one of the collateral vessels $150_{1..4}$ and direct the guidewire to approach and make contact with the distal cap 134 from the target vessel 140 in a retrograde approach. In some embodiments, similar to the proximal cap 132 previously described, the system may analyze the bending and deforming of the guidewire, the entry location in the intra-operative image data, force of the guidewire when the guidewire contacts the distal cap 134, etc., to determine an improved estimation of the stiffness, shape, and/or other properties of the distal cap 134. Based on such improved estimations from the intra-operative imaging data, the systems and methods may determine that the retrograde approach would be a very high level of difficulty to perform in the treatment procedure. The systems and methods may then adjust the treatment plan to terminate the treatment procedure (as neither an antegrade nor retrograde approach available), or reconsider the antegrade approach.

Further, the distal vessel may contain bifurcations near or inside the occlusion or may be damaged or diseased by the occlusion 130. Determination of the correct angles of the bifurcation direction and the health of the distal vessel 180 is important for crossing the occlusion with the guidewire. In the pre-operative imaging, these angles may be uncertain due to the presence of artifact (calcifications, blooming, etc.). In some embodiment, the intra-operative imaging data (e.g., X-ray images) provides improved visibility of the distal cap 134 and/or distal vessel 180 in comparison to the pre-operative imaging data, and such improved visibility enables the system to provide an improved estimation of such distal vessel properties. In some embodiments, captured intra-operative image data of the bending and deforming of the guidewire within the distal vessel 180 or difficulty advancing the guidewire through the distal vessel 180 may enable systems and methods to provide an improved estimation of such distal vessel properties. Based on such improved estimations from the intra-operative imaging data, the systems and methods may determine that the retrograde approach in the treatment plan would be a high level of difficulty to perform in the treatment procedure. The systems and methods may then adjust the treatment plan to terminate the treatment procedure (as neither an antegrade nor retrograde approach available), or reconsider the antegrade approach.

The characteristics of the occlusion 130 shown in Fig. 2 further include the occlusion length, course, and composition 170. Some embodiments may analyze the length of the occlusion 130, which is typically characterized as less than 20 millimeters (mm) versus great than or equal to 20 mm. Procedures performed on occlusion lesions with greater than or equal to 20 mm tend to have lower success rates and may be better treated with the treatment selection of the retrograde direction. Some embodiments may additionally or alternatively analyze the occlusion composition (e.g., calcium, blood clot, etc.) and the occlusion course (e.g., how the composition varies throughout the occlusion 130).

Actions performed during the treatment procedure may result in improved information on the properties of the occlusion length, composition, and course. For example, during the treatment procedure, a physician may insert a guidewire into the occlusion 130 past the proximal cap 132 (in antegrade direction) or past the distal cap 134 (in retrograde direction). In some embodiments, the system may analyze, from intra-operative imaging data, the bending and deforming of the guidewire, the location of the guidewire relative to the target vessel wall 14 as the guidewire progresses through the occlusion, force of the guidewire when the guidewire progresses through the occlusion, time to pass the guidewire through the occlusion 130, etc., to determine an improved estimation of the length, composition, and/or course of the occlusion 130.

In some embodiments, the cap ends of the occlusion 130 and the corresponding stump-angles may not be accurately determinable from the pre-operative image, causing an inaccurate estimation of the occlusion length. In some embodiment, the intra-operative imaging data (e.g., X-ray images) provides improved visibility of the occlusion 130 in comparison to the pre-operative imaging data, and such improved visibility enables the systems and methods to provide an improved estimation of one or more of these properties, such as providing improved visibility of the occlusion length or calcification. Further, from the multiple intra-operative images acquired of the progression of the treatment procedure, the method may better estimate the occlusion length. For example, when multiple intra-operative images are acquired of the occlusion 130 during the procedure, these images may be stacked (such as provided in Philips StentBoost) to obtain an image with improved signal-to-noise that allows improved determination of the occlusion length and course.

Based on such improved estimations from the intra-operative imaging data, in some embodiments, the systems and methods may determine that the crossing technique (e.g., through the lumen using a wiring technique) is not appropriate and adjust the treatment plan to a dissection and reentry technique, or may determine treatment is too difficult by the treatment procedure and adjust the treatment plan to terminate the treatment procedure.

In some embodiments, some or all of the properties of the proximal cap morphology, collateral circulation, quality of the distal vessel, distal cap morphology, occlusion length, occlusion course, and/or occlusion composition may be analyzed together to determine an adjustment to the treatment plan (such as adjustment to the approach direction treatment selection, terminate of the treatment procedure, etc.). For instance, in an example embodiments, the systems and methods may analyzed together the improved intra-operative estimations of a blunt shaped proximal cap, occlusion length greater than 20 mm, extreme calcification, and tortuosity collateral vessels to determine to adjust the treatment plan to terminate the treatment procedure (as neither an antegrade nor retrograde approach are appropriate).

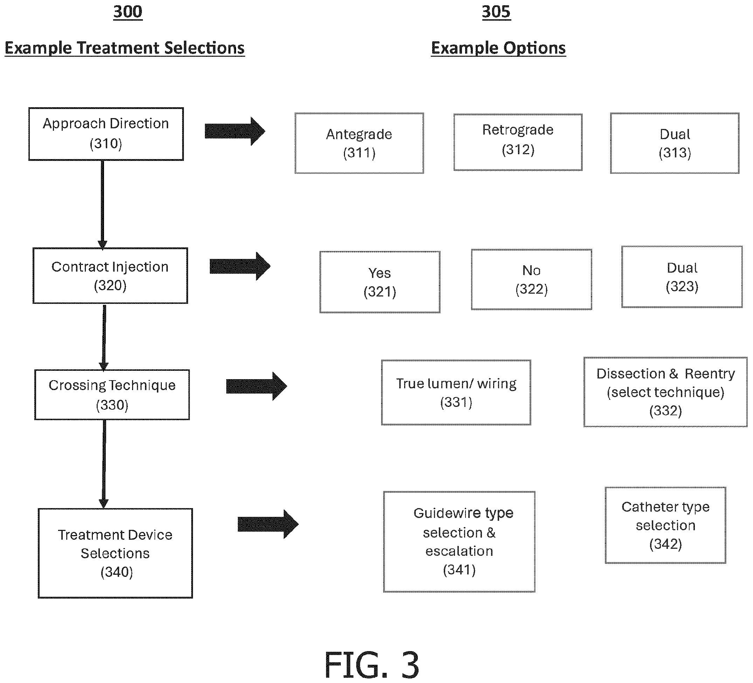

For illustrator purposes, the description of some figures provide examples of determining and adjusting the treatment selection of approach direction (e.g., antegrade and retrograde). The embodiments of the present disclosure are in no way limited to the treatment selection of approach direction. Other treatment selections including, but not limited to, the example treatment selections shown in Fig. 3 may also be incorporated without departing from the scope of the present teachings.

Fig. 3 illustrates a block diagram of example treatment selections for a treatment plan to treat occlusion 130, in accordance with some embodiments of the present disclosure.

As shown in Fig. 3, in some embodiments, the systems and methods may analyze the occlusion characteristics (e.g., treatment progress characteristics), such as shown in Fig. 2, to create and adjust example treatment selections 300 in a treatment plan according to the example treatment selection options 305. In the example shown in Fig. 3, the treatment selections 300 include approach direction 310 with the options of antegrade 311, retrograde 312, or dual antegrade-retrograde 313. In the example shown in Fig. 3, the treatment selections 300 also include contrast injection 320 with the options of yes 321, no 322, or dual 323 (i.e., inject into both antegrade and retrograde directions). In the example shown in Fig. 3, treatment selections 300 also include crossing technique 330 with the options of through the true lumen with wiring 331 or dissection and re-entry 332 (i.e., advance device inside the vessel wall but outside true lumen and re-enter into the lumen, such as by using a controlled antegrade retrograde tracking (CART) technique or subintimal tracking and reentry (STAR) technique. In the example shown in Fig. 3, the treatment selections 300 include treatment device selections 340 with the options of guidewire selection and escalation 341 (e.g., selecting a guidewire of particular size, shape, flexibility, etc. and escalating to guidewire(s) of smaller or large size(s), different shapes(s), flexibilities, etc. during progression of procedure) and catheter type selection 342 (e.g., balloon, laser, etc.).

The treatment selections shown in Fig. 3 are an example of some treatment selections included in embodiments of the present disclosure. Other treatment selections, without limitation, may also be incorporated without departing from the scope of the present teachings. For descriptions of example treatment selections included in some embodiments, refer to "Manual of Chronic Total Occlusion Interventions: A Step-by-Step Approach", Brilakis, Academic Press, Elsevier, London, UK, Chapter 21; 2022; "A Percutaneous Treatment Algorithm for Crossing Coronary Chronic Total Occlusion", Brilakis et al, JACC: Cardiovascular Interventions, The American College of Cardiology Foundation, Vol. 5, No. 2, ISSN: 1936-8798, 2012, pages 367-379; and "Guiding Principles for Chronic Total Occlusion Percutaneous Coronary Intervention: A Global Expert Consensus Document", Brilakis et al., Circulation, Vol. 140, No. 5, July 2019, page 420-433, which are all incorporated herein by reference.

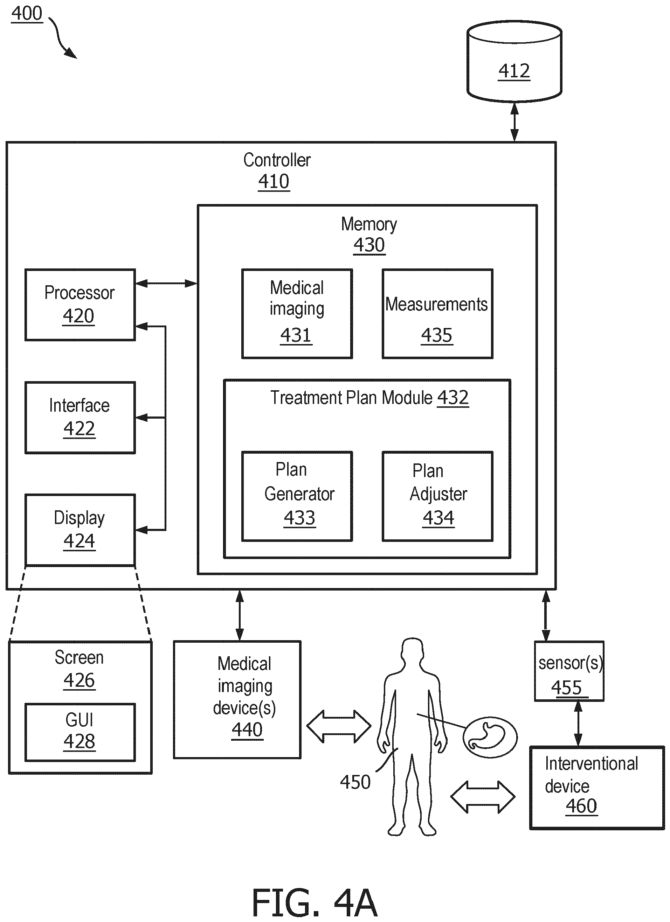

Fig. 4A illustrates a block diagram of a system 400 for creating and adjusting a treatment plan for a treatment procedure, in accordance with some embodiments of the present disclosure.

In Fig. 4A, system 400 includes a controller 410 that implements and manages processes described herein for creating and adjusting a treatment plan for a treatment procedure using medical images from one or more medical imaging devices 440, such as an X-ray imaging device, computed tomography (CT) imaging device, or an ultrasound imaging device, for example. The controller 410 includes processor 420, memory 430, user interface 422 and display 424. The processor 420 communicates with the one or more medical imaging devices 440 through an imaging interface (not shown). In some embodiments, the one or more medical imaging devices 440 include an X-ray imaging device, such as a fixed or mobile C-arm X-ray device, with an X-ray source configured to generate X-ray radiation and an X-ray detector configured to acquire X-ray images of the vasculature of the subject 450. In some embodiments, the one or more medical imaging devices 440 include a CT imaging device that obtains CT images of the vasculature of the subject 450. In some embodiments, the one or more medical imaging devices 440 include an ultrasound device that obtains ultrasound images of the vasculature of the subject 450, such as an intravascular ultrasound (IVUS) probe, intracardiac echocardiography (ICE) probe, Transesophageal echocardiography (TEE), etc. Other types of interventional imaging devices may also be incorporated without departing from the scope of the present teachings.

The processor 420 in Fig. 4A also communicates with one or more sensors 455 disposed on or in proximity to an interventional device 460 insertable into the subject 450 for performing the treatment. The processor 420 may communicate with the one or more sensors 455 through a sensor interface (not shown). In embodiments, the one or more sensors 455 are configured to measure various properties related to the interventional device 460, such as force applied by the interventional device when guided through the anatomy (e.g., blood vessels) of the subject 450 or when contacts the anatomy of the subject (e.g., occlusion with the blood vessels of the subject 450). The interventional device 460 may include any device insertable into the body of the subject 450 for medical treatment, such as a guidewire, catheter, imaging device, etc.

The memory 430 stores instructions executable by the processor 420. When executed, the instructions cause the processor 420 to implement one or more processes for creating and adjusting a treatment plan using medical images (e.g., interventional images) acquired by the one or more medical imaging devices 440 and measurements acquired by the one or more sensors 455. The medical images and/or measurements may be provided from the one or more medical imaging devices 440 and/or the one or more sensors 455 in real-time or near real-time during the treatment procedure, or may be retrieved from storage (e.g., database 412). Other treatment data may also be retrieved by the processor 420 from the database 412, such as the treatment plan for the subject 450, personal data of the subject 450 (e.g., age, weight), historical treatment data of the subject 450 (e.g., disease history), properties of the interventional device (e.g., size, flexibility), etc. For purposes of illustration, the memory 430 is shown to include software modules, each of which includes sets of instructions, executable by the processor 420, corresponding to an associated capability of the system 400.

The processor 420 is representative of one or more processing devices, and may be implemented by a general purpose computer, a central processing unit (CPU), a digital signal processor (DSP), a graphical processing unit (GPU), a tensor processing unit (TPU), a computer processor, a microprocessor, a state machine, programmable logic device, field programmable gate arrays (FPGAs), application specific integrated circuits (ASICs), or combinations thereof, using any combination of hardware, software, firmware, hard-wired logic circuits, or combinations thereof. Any processor or processing unit herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices. The memory 430 and/or the database 412 may also refer to storage within a single storage device local to the processor 420, a single storage device remote (cloud-based) from the processor 420, or distributed among multiple computer systems.

The memory 430 is representative of one or more memories, and may include main memory and/or static memory, where such memories may communicate with each other and the processor 420 via one or more buses. The memory 430 may be implemented by any number, type and combination of random access memory (RAM) and read-only memory (ROM), for example, and may store various types of information, such as software algorithms, machine-learning models, and computer programs, all of which are executable by the processor 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 430 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 430 may store software instructions and/or computer readable code that enable performance of various functions. The memory 430 may be secure and/or encrypted, or unsecure and/or unencrypted.

The system 400 may also use database 412 for storing information that may be used by the various software modules of the memory 430. For example, the database 412 may include historical data from previous treatment of the subject 450 and/or of other similarly situated subjects, having treatment for an occlusion in a blood vessel. The historical data may include historical treatment images, historical treatment measurements, historical treatment plan selections, historical treatment outcomes, etc. from previous treatment procedures performing on the subject 450 or similarly situated subjects. In some embodiments, the stored data may be used for scoring the condition of the subject 450 (e.g., occlusion) and/or treatment selections for treating the subject 450. In some embodiments, the stored data may be used for training a machine-learning model (algorithm), such as a neural network, for example, as discussed below.

The database 412 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 412 comprises tangible storage mediums for storing data and executable software instructions and is non-transitory during the time data and software instructions are stored therein. The database 412 may be secure and/or encrypted, or unsecure and/or unencrypted. For purposes of illustration, the database 412 is shown as a separate storage medium, although it is understood that it may be combined with and/or included in the memory 430, without departing from the scope of the present teachings.

The user interface 422 is configured to provide information and data output by the processor 420, the memory 430, the one or more medical imaging devices 440, and/or the one or more sensors 455 to the user (e.g., physician) and/or for receiving information and data input by the user. That is, the user interface 422 enables the user to enter data and to control or manipulate aspects of the processes described herein, and also enables the processor 420 to indicate the effects of the user's input, which may include control or manipulation of the one or more medical imaging devices 440 and/or interventional device 460. All or a portion of the user interface 422 may be implemented by a graphical user interface (GUI), such as GUI 428 viewable on the display 424, discussed below. The user interface 422 may include one or more interface devices, such as a mouse, a keyboard, a trackball, a joystick, a microphone, a video camera, a touchpad, a touchscreen, voice or gesture recognition captured by a microphone or video camera, for example.

The display 424 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 424 includes a screen 426 for viewing medical images and/or measurements of the subject 450, along with various features described herein to communicate to the user current treatment selections of a treatment plan, recommendations regarding updating the treatment plan, recommended adjustments to the treatment selections of treatment plan, current interventional device 450 selected for performing treatment plan, recommended adjustments to interventional device for performing treatment plan, etc. The screen 426 further enables viewing of the GUI 428 to enable the user to interact with the displayed images and features.

Referring to the memory 430, the various modules therein store sets of data and instructions executable by the processor 420 to create and determine an adjustment to the treatment selections in the treatment plan, as mentioned above. Medical imaging module 431 is configured to receive and process medical images for the treatment of the subject 450. The medical images may be acquired non-invasively or invasively by the one or more medical imaging devices 440, and may be received from the one or more medical imaging devices 440 in real-time or near real-time during a contemporaneous imaging session of the subject 450. For example, the medical images may be intra-procedural angiography images acquired during an interventional procedure (e.g., PCI). Alternatively, or in addition, the medical images may be previously acquired images (e.g., pre-operative CCTA images), obtained during previous imaging session(s), which have been retrieved from storage (e.g., database 412). The medical images may show the interventional device being guided through the vessels of the subject 450 and making contact with an occlusion in a vessel of the subject 450, and may be displayed on the display 424.

Measurements module 435 is configured to receive and process measurements from the one or more sensors 455 and other data from the database 412 for the treatment of the subject 450. The measurements may be acquired non-invasively or invasively by the one or more sensors 455, and may be received from the one or more sensors 455 in real-time or near real-time during a contemporaneous imaging session of the subject 450. For example, the measurements may be intra-operative measurements acquired during progression of an interventional procedure. Alternatively, or in addition, the measurements may be previously acquired measurements, obtained during previous treatment procedure, which have been retrieved from storage (e.g., database 412). The measurement may indicate properties of the interventional device 460 as the interventional device 460 is guided through the vessels of the subject 450 and/or inserted into an occlusion in a vessel of the subject 450, and may be displayed on the display 424. The measured properties may be force applied by the interventional device 460, positioning (e.g., bending and deforming) of the interventional device 460, motion of the interventional device 460, etc.

Treatment plan module 432 in the memory 430 includes instructions configured to analyze the medical images, measurement, and/or other data of the subject 450, in order to create a treatment plan of the subject 450, determine that that the treatment plan of the subject 450 needs adjusting during progression of a treatment procedure, and/or recommend adjustments to a treatment selection in the treatment plan during progression of the treatment procedure. The treatment plan module 432 may be organized as a plan generator module 433 and a plan adjuster module 434.

The plan generator module 433 includes instructions to create an initial treatment plan with first order treatment selection, such as from pre-operative imaging data (e.g., CCTA) acquired by a medical imaging device (e.g., CT imaging device) of the one or more medical imaging devices 440. The plan generator module 433 may include instructions for analyzing the pre-operative imaging data and/or other treatment data of the subject 450, such as personal data of the subject 450 accessed from the database 412. The plan generator module 433 may include instructions to extract from the pre-operative imaging data and analyze treatment progress characteristics, including characteristics related to (1) the proximal cap morphology of an occlusion within a vessel of the subject 450, (2) the occlusion length, course, and composition, (3) quality of the vessel distal to the occlusion, and/or (4) the collateral circulation, to create the initial treatment plan.

The plan adjuster module 434 includes instructions to determine whether the initial treatment plan requires adjustment during the interventional procedure and, if so, determine the adjustments to specific treatment selections in the treatment plan. The plan adjustment module 434 may include instructions for analyzing intra-operative imaging data (e.g., X-ray images) acquired by a medical imaging device (e.g., X-ray imaging device) of the one or more medical imaging devices 440, measurements acquired by the one or more sensors 455, and/or other treatment data of the subject 450, such as personal data of the subject 450 accessed from the database 412. The plan adjuster module 434 may include instructions to extract from the pre-operative imaging data and analyze treatment progress characteristics, including characteristics related to (1) the proximal cap morphology of an occlusion within a vessel of the subject 450, (2) the occlusion length, course, and composition, (3) quality of the vessel distal to the occlusion, and/or (4) the collateral circulation, for adjustment of the treatment plan.

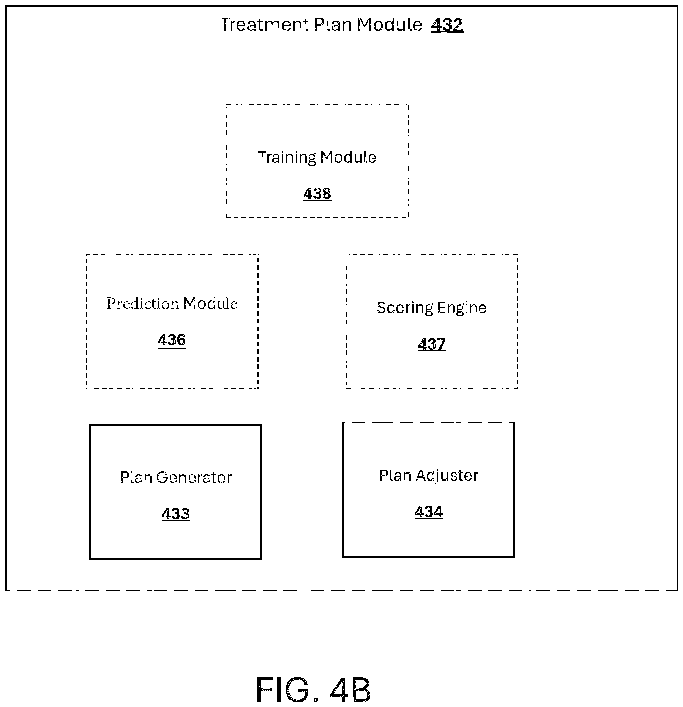

Fig. 4B illustrates a block diagram of an example treatment plan module included in some embodiments of the system in Fig. 4A. The module of Fig. 4B is an example of the treatment plan module 432 that includes the plan generator module 433 and plan adjuster module 434, as previously described with respect to Fig. 4A, and also the optional prediction module 436, scoring engine 437, and training module 438, in accordance with some embodiments of the present disclosure. In some embodiments, a subset of the prediction module 436, scoring engine 437, and training module 438 are included in the treatment plan module 432. For example, the treatment plan module 432 may only include the scoring engine 437 and prediction module 436 (and the training module 438 may be configured and executed on a different computer system).

The scoring engine 437 may include instructions to score one or more properties of the treatment progress characteristics of the subject 450 extracted from the images of the subject 450 to determine adjustments to the treatment plan. For example, the instructions may analyze an occlusion being treated and assign scores indicating the level of difficulty that the properties of the treatment progress characteristics cause to achieving a successful treatment outcome of the occlusion. For example, a high amount of calcification in the occlusion causes the treatment procedure to be more difficult to perform, so the assigned difficulty score for the occlusion composition characteristic would be set high. In embodiments, the scoring engine 437 may determine the score by application of anatomical rules defined for occlusion characteristics, which are stored and accessed from database 412, to intra-operative images; comparison of the inter-operative images to scored historical images (in database 412) which include scores for the occlusion characteristics depicted in the historical images, and/or communication with prediction module 436 trained from such historical images to predict scores for the occlusion progression characteristics. In some embodiments, the scoring engine 437 may execute known image processing or computer vision to compare the inter-operative images to the anatomical rules and/or historical images.

The scoring engine 437 may also include instructions that calculate a total treatment difficulty score for the treated occlusion by combining the difficulty scores assigned to the treatment progress characteristics of the occlusion. As an improved assessment of the treatment progress characteristics may be made based on actions performed during the treatment procedure, such as inserting a guidewire into the occlusion, the scoring of the treatment progress characteristics may change during progression of the procedure (and thereby cause the treatment difficulty score for the occlusion to change compared to a previously calculation of the treatment difficulty score) indicating a need to adjust the treatment plan.

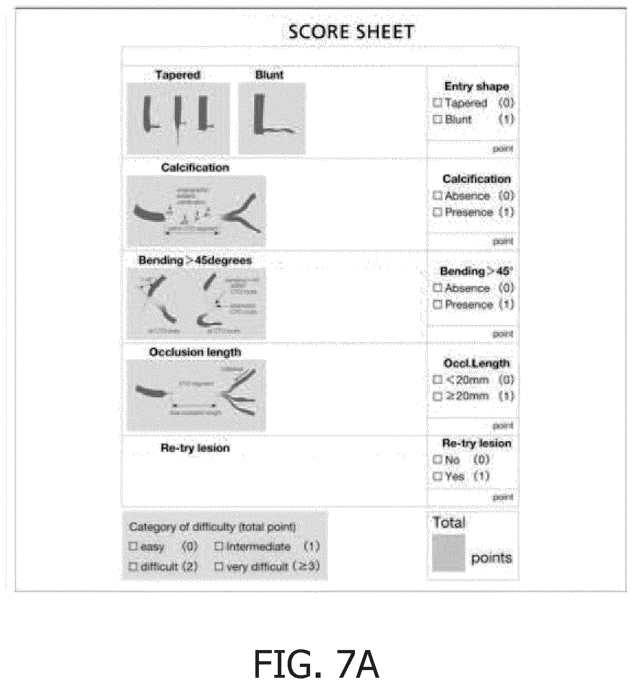

The plan adjuster module 434 may include further instructions to determine whether the treatment plan requires adjustment during the interventional procedure according to the change in the treatment difficulty score calculated by the scoring engine 437. For example, one or more threshold scores may be defined and stored which correspond to one or more categories of difficulty of the treatment procedure for treating the occlusion (such as shown in Fig. 7A). If the current treatment difficulty score meets a different defined threshold score than the previous calculation of the treatment difficulty score, the plan adjuster module 434 may determine that an adjustment to the treatment plan is required, such as adjusting a treatment selection in the treatment plan or adjusting the treatment plan to terminate the treatment procedure (as the treatment procedure is determined to be too difficult to proceed with any treatment selection).

The prediction module 436 of Fig. 4B includes a machine-learning model (e.g., neural network) that is trained by historical images of a treatment.

The processor 420 may include or have access to the prediction module 436, which may be implemented as software that provides artificial intelligence (e.g., machine-learning models) and applies machine-learning described herein. The prediction module 436 may reside in any of various components in addition to or other than the processor 420, such as the memory 430, the database 412, an external server, and/or the cloud, for example. When the prediction module 436 is implemented in a cloud (not shown), such as at a data center, for example, the prediction module 436 may be connected to the processor 420 via the internet or other communication network using one or more wired and/or wireless connection(s).

The prediction module 436 includes instructions that input data to one or more trained machine-learning model. The data may include the treatment plan and treatment data, such as medical images from the one or more medical imaging devices 440, treatment progress characteristics extracted from the images, measurements from the one or more sensors 455, and/or other input data related to the treatment (e.g., stored in the database 412). From the input data, the trained model may predict and output whether the treatment plan requires adjustment and/or the specific adjustments to one or more treatment selections in the treatment plan. In some embodiments, the machine-learning model may implemented as neural network, for example, such as an artificial neural network (ANN), a convolutional neural network (CNN), a recurrent neural network (RNN), variational autoencoder (VAE), a transformer, or a U-net model.

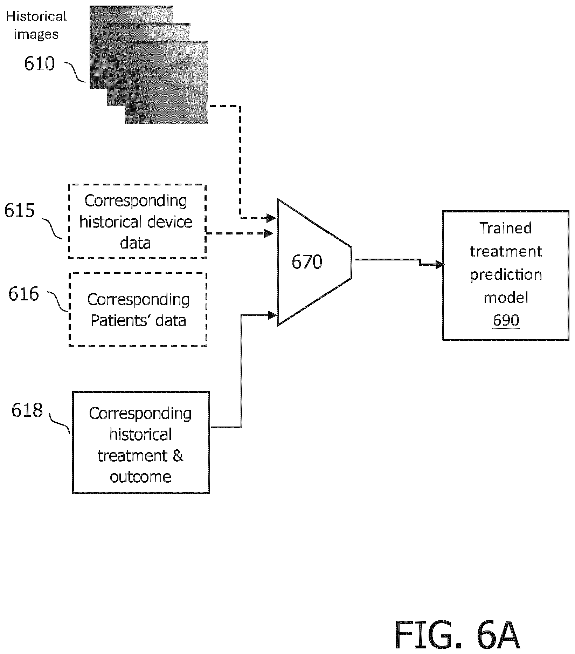

The training module 438 of Fig. 4B includes instructions to perform training of the machine-learning model based on historical data from past treatment procedures (e.g., treating an occlusion in a blood vessel) of past patients or possibly the same patient during past treatment procedures. The historical data may include pre-operative and/or intra-operative images that include image features of the past patients' anatomies, such as treatment progress characteristics related to occlusions within the past patients, for example morphology of the occlusion proximal cap, distal cap, distal vessel, and/or collateral vessels. The historical data may also include treatment plans corresponding to the past treatment procedures, treatment devices used in the past treatment procedures, personal metrics of the patients (e.g., age, weight, height, health, etc.), and/or outcomes of the past treatment procedures. The training may include training the machine-learning model to predict the probability of successful outcome of different treatment selections (such as probability of successful outcome of antegrade approach and of retrograde approach) and selecting the treatment selection with the highest predicted probability of success. The training may also include training the machine-learning model to predict termination of treatment procedure when the probability of successful outcome is below a predetermined threshold (e.g., less than 50%). In some embodiments, the predicted probability of successful outcome is determined as a score indicating difficulty of the treatment procedure or treatment selections in the treatment plan for the treatment procedure. The machine-learning model may be trained in a supervised, or in some cases unsupervised, semi-supervised, or reinforced manner, to implement the operations that are performed by the processor 420. In some embodiments, the machine-learning model is trained in accordance with the embodiments described with respect to Fig. 6A.

In some embodiments, the training module 438 may be configured as part of the treatment plan module 432 in memory 430 of the controller 410 (as shown in Fig. 4B). In other embodiments, the training module 438 may be configured in memory of a different computer system that may or may not be in communication with the controller 410 of Fig. 4A. In embodiments in which the training module 438 is not in communication with the controller 410 of Fig. 4A, the trained machine-learning module generated by the training module 438 may be copied to memory 430 or database 412 for access by the prediction module 436.

The prediction module 436 is configured to apply the trained machine-learning model to current input data from the subject 150, including the current treatment plan, to predict whether the treatment plan requires adjustment and/or treatment selections in the treatment plan that require adjustment. The input data may include an image showing the occlusion in the subject's vasculature. The prediction module 436 may extract image features (e.g., treatment progress characteristics related to the occlusion) from the medical image data that are used by the prediction module 436 to evaluate properties related to the image features, including using known image processing techniques. The image features may include properties of the proximal cap of the occlusion (e.g., stiffness, shape), collateral vessels (tortuosity), etc. The image features extracted may depend on the imaging modality, quality of the images, actions being performed to the occlusion (e.g., insertion of guidewire into proximal cap). Optionally, the input data may further include device information of the device inserted into the occlusion, including measurements taken by a sensor on the device, and/or patient information that describes characteristics of the subject 450. For example, the device information may include the diameter, material and flexibility of the device and force measured by a sensor on the device when the device is inserted into the proximal cap. The patient data, such as patient age, blood pressure, weight, heart health, smoking history, and family health history, for example.

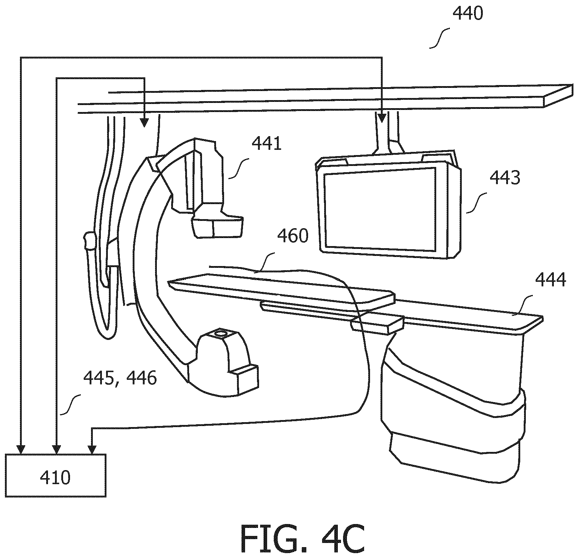

Fig. 4C illustrates a schematic diagram of an example C-arm medical imaging device included in some embodiments of the system in Fig. 4A.

The medical imaging device 440 includes an angiographic imaging component (e.g., C-arm) 441 for providing angiographic image data 445, 446 of a patient; a patient bed 444; a monitor 443 for displaying the data outputted by the processor 420 of the controller 410, such as graphical representations of the angiographic image data 445, 446, and a user input device such as a keyboard, a mouse, a touchscreen, and so forth, and which is configured to receive user input relating to operations performed by the processor 420. The medical imaging device 440 images the region of the patient including the occlusion 130 and the interventional device 460 (e.g., guidewire) performing the treatment procedure on the occlusion 130.

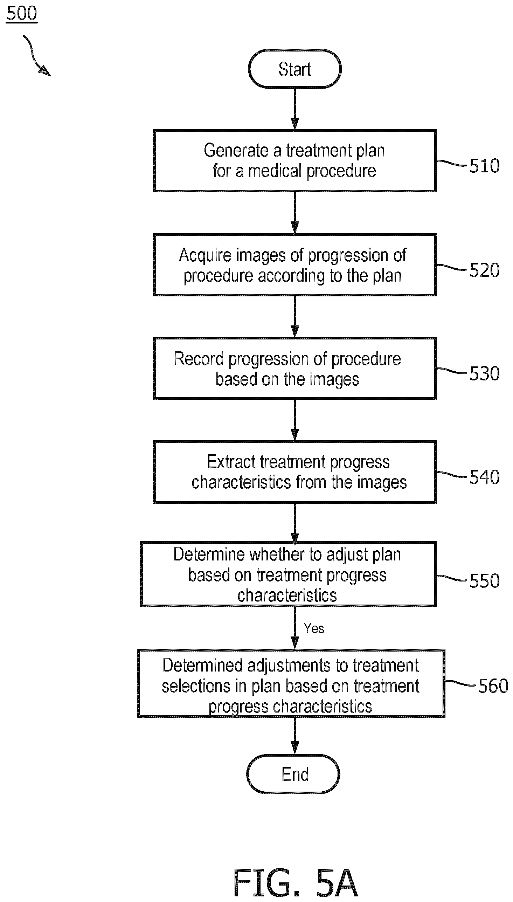

Fig. 5A illustrates a flow diagram of a computer-implemented method 500 for creating and adjusting a treatment plan for a treatment procedure, in accordance with some embodiments of the present disclosure. In some embodiments, the method 500 of Fig. 5A may be stored as instructions 430 and executed by the processor 420 of the control system 400 shown in Figs. 4A-4C.

The method 500 starts, in block 510, by generating the treatment plan for a medical procedure. The method 500 generates the treatment plan to treat a condition of an anatomical structure, such as an occlusion (e.g., CTO) in a blood vessel (e.g., coronary artery), by the medical procedure, such as a percutaneous coronary intervention (PCI). The method 500 may generate the treatment plan to include one or more first order treatment selections for treatment of the occlusion determined based on pre-operative imaging data (e.g., CCTA). For example, the method 500 may include selection of antegrade direction to approach the occlusion base on the pre-operative imaging data. The method 500 may generate the treatment plan based on assessment of characteristics related to the anatomical structure in the pre-operative imaging data. In some example embodiments, the method 500 generates the treatment plan based on analysis of treatment progress characteristics, including (1) proximal cap morphology of an occlusion, (2) occlusion length, course, and composition of the occlusion, (3) quality of the vessel distal to the occlusion, and/or (4) characteristics of the collateral circulation in the anatomical structure.

The method 500, in block 520, acquires intra-operative data, including intra-operative images of the progression of the medical procedure performed according to the treatment plan. In some embodiments, the intra-operative images are X-ray images captured by an X-ray imaging device, such as medical imaging device 440 shown in Fig. 4A or 4C. The intra-operative images may include an anatomical structure (e.g., coronary artery), the treated condition of the anatomical structure (e.g., occlusion in the coronary artery), etc. The intra-operative images may also include an interventional device 460 (e.g., guidewire, catheter, intravascular ultrasound (IVUS), intracardiac echocardiography (ICE)), for treating the condition. In some embodiments, the intra-operative images capture actions performed during the treatment procedure in relation to the occlusion, such as insertion of the interventional device 460 into the occlusion. In some embodiments, the intra-operative data includes measurements by a sensor (e.g., sensor on or near the device 460) related to the actions performed during the treatment procedure in relation to the occlusion. In some embodiments, the intra-operative data includes other data stored or collected in relation to the treatment (e.g., data related to the health of the patient).

The method 500, in block 530, records the progression of the medical procedure based on the intra-operative imaging data.

The method 500, in block 540, extracts treatment progress characteristics based on analysis of the intra-operative images acquired of the progression of the medical procedure. In some embodiment, the method 500 continuously extracts and updates the treatment progress characteristics based on the analysis of the intra-operative images continuously acquired during the procedure. In some embodiments, the treatment progress characteristics include (1) the proximal cap morphology of an occlusion, (2) occlusion length, course, and composition, (3) quality of the vessel distal to the occlusion, and/or (4) the collateral vessels in the anatomical structure.

In some embodiments, the actions performed during the treatment procedure result in improved information related to the treatment progress characteristics, which is captured in the intra-operative images acquired of the progression of the medical procedure. Example of actions that provide such improved information are described with respect to Fig. 2.

For one such example, a guidewire may be advanced to approach the proximal cap 132 of the occlusion in the antegrade direction during progression of the medical procedure. The guidewire may bend and deform in a particular manner as the guidewire contacts the proximal cap of the occlusion and/or may enter the occlusion close to the vessel wall. This actions of the guidewire may be captured in the intra-operative images and provide improved information on the extracted treatment progress characteristics. For example, the actions may provide improved information related to the proximal cap of the occlusion (such as its stiffness) than was previously known from the pre-operative imaging data.

In some embodiments, the method 500 provides the user (e.g., physician) instructions to execute actions (e.g., tests) during the treatment procedure. The method then analyzes the intra-operative images acquired during the instructed actions with the knowledge of the actions the user was performing in the acquired intra-operative images. For example, rather than analyzing all encounters of the guidewire with the proximal cap of the occlusion to determine the proximal cap morphology, the method 500 may instruct the user to approach the proximal cap with the guidewire in a predefined way (e.g., at a particular angle, at a particular location, with a particular force, etc.) and acquire images during the instructed approach to the proximal cap. The method 500 may then analyze the treatment progress characteristics extracted from these images using the knowledge of the predefined way the proximal cap for improved insight of the occlusion. Further, in some embodiments, an improved assessment of the treatment progress characteristics may be performed from the intra-operative images acquired due to the improved visuality of the occlusion captured by the intra-operative imaging modality (e.g., X-ray) than the pre-operative imaging modality (e.g., CT).

The method 500, in block 550, determines whether to adjust the treatment plan based on the treatment progress characteristics. In some embodiments, the method 500 scores the pre-operative images from block 520 and/or extracted treatment progress characteristics from block 540 to determine whether the treatment plan needs to be adjusted.

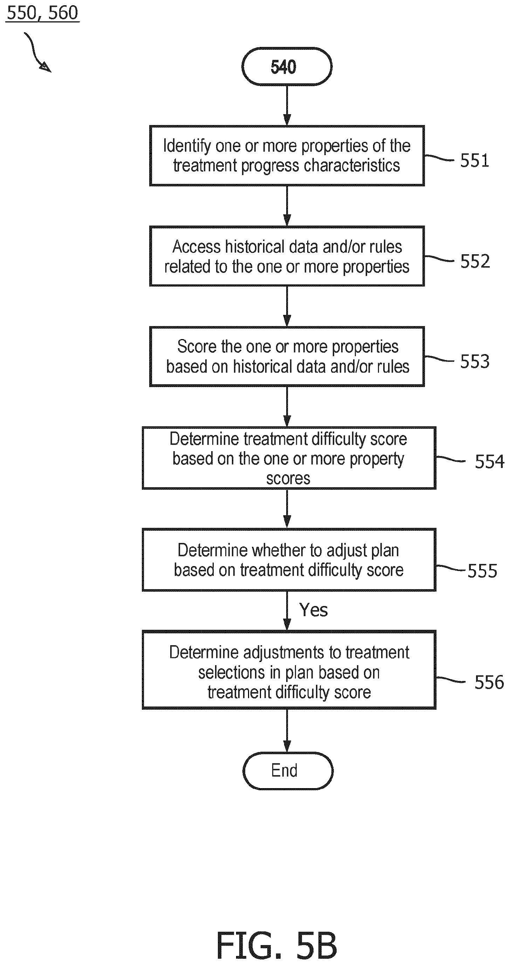

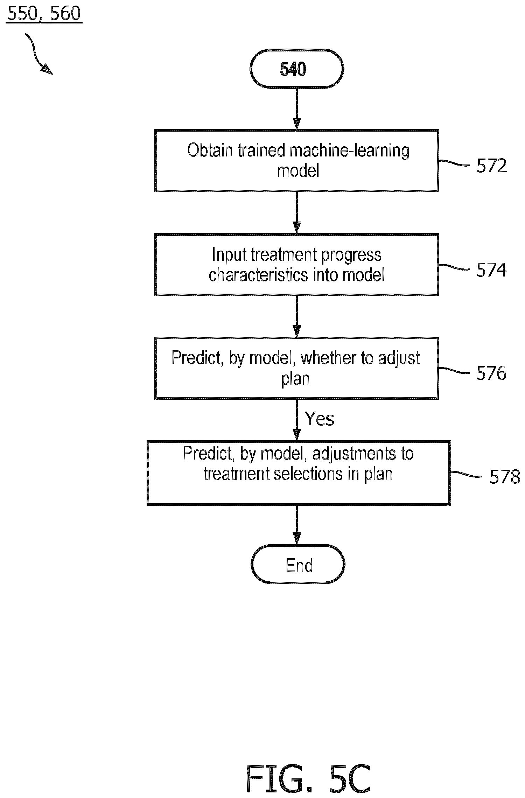

Examples of the use of scoring to determine whether the treatment plan needs to be adjusted are described with respect to the scoring engine 437 of Fig. 4B and the method of Fig. 5B. In some embodiments, the method 500 inputs the pre-operative images from block 520 and/or extracted treatment progress characteristics from block 540, together with the preoperative images from block 510, into a trained model or algorithm, such as a trained machine-learning model or algorithm. Such model analyzes the input based on historical occlusion treatment data and predicts whether the treatment plan needs to be adjusted. Examples of use of such model to predict whether the treatment plan needs to be adjusted are described with respect to the prediction module 436 of Fig. 4B and the method of Fig. 5C.

If the method 500, in block 550, determines to adjust the treatment plan, the method 500, in block 560, determines adjustments to the treatment selections in the treatment plan. In some embodiments, the method scores the pre-operative images from block 520 and/or extracted treatment progress characteristics from block 540 to determine adjustments to one or more treatment selections in the treatment plan. Examples of the use of scoring to determine adjustments to one or more treatment selections in the treatment plan are described with respect to the scoring engine 437 of Fig. 4B and the method of Fig. 5B. In some embodiments, the method inputs the pre-operative images from block 520 and/or extracted treatment progress characteristics from block 540, together with the preoperative images from block 510, into a model or algorithm, such as a trained machine-learning model or algorithm, which analyzes the input to predict adjustments to one or more treatment selections in the treatment plan. Examples of use of such model to predict adjustments to one or more treatment selections are described with respect to the prediction module 436 of Fig. 4B and the method of Fig. 5C.

In some embodiments, when the method 500 determines a need to adjust the treatment plan in block 550, the method 500 may output an indication to the user (e.g., physician) recommending adjustment of the treatment plan, such as via display 424 of Fig. 4A or monitor 443 of Fig. 4C. The output may include recommendation of another treatment strategy for the treatment plan, which may include recommendation of specific adjustments to the treatment selections in the treatment plan determined in block 560. In some embodiments, the output may further include an assessment of the treatment progress characteristics and properties thereof based on the intra-operative imaging data and/or pre-operative imaging data, such as outputting the determined stiffness and shape of the proximal cap. In some embodiments, if the method 500 determines a need to adjust the treatment plan, the method 500 may automatically adjust the treatment plan, which may include automatically adjusting the treatment selections in the treatment plan.

Fig. 5B illustrates a flow diagram of a computer-implemented method for scoring characteristics related to an occlusion to determine adjustment of a treatment plan, in accordance with some embodiments of the present disclosure. The method of Fig. 5B provides an example method for executing blocks 550 and 560 of the method in Fig. 5A. In some embodiments, the method of Fig. 5B is stored in memory 430 executed by processor 420 of the control system 400 shown in Figs. 4A-4C.

The method of Fig. 5B starts, in block 551, by identifying one or more properties of the treatment progress characteristics extracted from imaging data, such as the intra-operative images of block 520 of Fig. 5A and/or the pre-operative images of block 510 of Fig. 5A. Such properties may include the shape of the proximal cap of the occlusion, the stiffness of the proximal cap of the occlusion, the length of the occlusion, the quality of the distal vessel of the occlusion, the circulation of the collateral vessels, the tortuosity of the collateral vessels, and so forth.

The method, in block 552, accesses historical data and/or anatomical rules related to the properties of the treatment progress characteristics. The rules may be stored in the database 412 and include instructions on how to score different properties of the treatment progress characteristics of the occlusion. The method, in block 553, scores the properties of the treatment progress characteristics based on the historical data and/or rules from the database 412.

In some embodiments, the method applies the rules to properties of the treatment progress characteristics and assign scores indicating the difficulty that the properties cause for treating the occlusion. Occlusions with properties causing high difficulty for treatment may not be suitable for treatment by a treatment procedure (e.g., PCI). The method may apply rules that include action performed during the treatment procedure, such as bending or deforming of an inserted guidewire. For example, the rules may include a rule for the shape of a proximal cap of an occlusion, which assigns a score of "1" for a blunt shaped proximal cap or a score of "0" for a tapered shaped proximal cap, as entering a blunt shaped proximal cap with an interventional device is of high difficulty to perform. The method may analyze the shape of the proximal cap based on an intra-operative image of the bending or deforming of the inserted guidewire (such as by using a known image processing technique) and assign the shape score to the proximal cap according to this rule. For another example, the rules may include a rule for length of the occlusion, which assigns a score of "0" if less than 20 mm or "1" if greater than or equal to 20 mm, as a longer occlusion is of higher difficulty to treat. The method may analyze the occlusion length extracted from an image acquired during progression of the procedure (such as by using a known image processing technique) and assign a score to the length according to this rule. A non-limiting example of scores to such occlusion properties is illustrated in Fig. 7A.

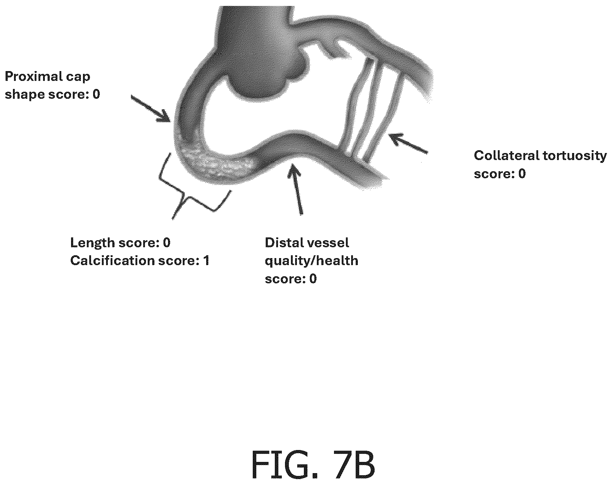

In some embodiments, the historical data may be used to score the occlusion. The historical data may comprise historical images of occlusions in blood vessels of patients. Properties related to the occlusions in the historical images may be annotated with a score. In some embodiments, the scores may be included in the historical images (e.g., as annotations). Fig. 7B illustrates an example of a historical image with properties related to an occlusion annotated with such score. In the example historical image in Fig. 7B, the proximal cap has a tapered shape annotated with the score of "0"; a length less than 22 mm annotated with "0"; high calcification annotated with the score of "1"; good distal vessel quality (no damage) annotated with a score of "0"; and little collateral tortuosity annotated with a score of "0".

The method may apply a known imaging processing technique or computer vision to compare the treatment progress characteristics in the historical images and the current intra-operative images. For example, the method may compare a proximal cap extracted from an intra-operative image to the proximal caps in the set of historical images and select the historical image with the closest matching proximal cap. The method may then assign to the proximal cap extracted from the current image the score associated with the proximal cap in the matching historical image. In some examples, the historical images may include actions performed to the proximal cap, such as insertion of an interventional device (e.g., guidewire) into the proximal cap, and shows the bending and/or deforming caused by the insertion. The current intra-operative images may also capture the insertion of a guidewire into an occlusion. The comparison by the method may include the comparison of the bending and/or deforming of the guidewires in the current images and the historical images.

In some embodiments, the method uses both the rules and the historical images to assign a score to properties of the extracted treatment progress characteristics. For example, the length of the occlusion may be evaluated by on a rule to assign a length and the proximal cap may be compared to historical images to assign a proximal cap score.

The method, in block 554, determines a treatment difficulty score based on the scores assigned to different properties of the treatment progress characteristics. The method may calculate the treatment difficulty score for the treatment plan by combining each of the property scores for the occlusion. The method may also calculate a treatment difficulty score for a particular treatment selection by combining each of the property scores relevant to that treatment selection. For example, in some embodiments, certain properties (e.g., proximal cap shape, proximal cap stiffness) may be considered relevant to determine the difficulty of an antegrade approach to the occlusion. In these embodiments, the method would combine the difficulty score of these relevant properties to calculate the treatment difficulty score for the antegrade approach. In some embodiments, the method may calculate a treatment difficulty score for a current treatment selection in the treatment plan and also alternative treatment selection. For example, the method may calculate a treatment difficulty score for the antegrade approach (which is the current treatment selection in the treatment plan) and also for the alternative treatment selections of a retrograde approach and a dual antegrade-retrograde approach.

The method, in block 555, determine whether to adjust the treatment plan based on the treatment difficulty score. In some embodiments, one or more threshold scores may be defined that indicates different levels of difficulty of the treatment procedure for treating the occlusion, such as from easy to very difficult (as shown in Fig. 7A). In some embodiments, the difficulty score calculated for a current set of images may be compared to the one or more threshold scores to determine the level of difficulty of the treatment.

As an improved assessment of the treatment progress characteristics may be made based on actions performed during the treatment procedure, such as inserting a guidewire into the occlusion, the scoring of the treatment progress characteristics may change during progression of the procedure (and thereby cause the treatment difficulty score for the occlusion to change compared to a previously calculation of the treatment difficulty score). If the treatment difficulty score calculated for the treatment progress characteristics in a current set of intra-operative images meets a different threshold than the treatment difficulty score calculated for a previous set of images, the method may recommend to adjust the treatment plan. For example, if the score of a current image increased to a higher threshold level, the method may determine to analyze whether an adjustment to a treatment selection in the plan may decrease the treatment difficulty score (such as an adjustment from an antegrade to retrograde approach). For another example, if the score of the current image now exceeds an extreme difficulty threshold level, the method may recommend to stop the treatment procedure.

The method, in block 556, determines adjustments to the treatment selections in the plan based on the treatment difficulty score. In some embodiments, one or more threshold scores may be defined for a particular treatment selection. For example, threshold scores may be defined for the difficulty of the approach to the occlusion. If the score for the antegrade approach calculated from a set of current intra-operative image increases to a new difficulty threshold and the score for the retrograde approach calculated from the set of current intra-operative images is now below than the score for the antegrade approach, the method may determine an adjustment to change the treatment selection from antegrade to retrograde. For example, improved assessment of proximal cap and collateral vessels in the current set of images may indicate that the proximal cap is stiffer and the collateral vessels less tortuosity than originally assessed from the pre-operative imaging data, thereby causing the difficulty score for the antegrade approach to exceed the difficulty score for the retrograde approach.

Fig. 5C illustrates a flow diagram of a computer-implemented method for predicting adjustment of a treatment plan using a machine-learning model, in accordance with some embodiments of the present disclosure. The method of Fig. 5B provides an example method for executing blocks 550 and 560 of the method in Fig. 5A. In some embodiments, the method of Fig. 5B is stored in memory 430 executed by processor 420 of the control system 400 shown in Figs. 4A-4C.

The method of Fig. 5C, in block 572, obtains a trained machine-learning model that is configured to predict whether a treatment plan needs adjustment and, if so, the treatment selections to adjust in the treatment plan. In some embodiments, the machine-learning model is trained according to a method described with respect to Fig. 6A.

The method, in block 574, input treatment progress characteristics into the trained machine-learning model. In other embodiments, the treatment progress characteristics are extracted from the images and input into the machine-learning model. In some embodiments, the treatment progress characteristics include one or more of (1) the proximal cap morphology of an occlusion, (2) the occlusion length, course, and composition, (3) the quality of the vessel distal to the occlusion, and/or (4) the collateral circulation In some embodiments, the treatment progress characteristics are included in intra-operative and/pre-operative images of an occlusion and the images are input into the machine-learning model. The machine-learning model may then extract the treatment progress characteristics in the images. The extraction may include extraction of properties the treatment progress characteristics, such as occlusion proximal cap properties (e.g., location, shape, stiffness), occlusion distal cap properties (e.g., location, shape, stiffness), vessel distal to the occlusion properties (e.g., location, size, damage), occlusion collateral vessels properties (location, tortuosity, angle to vessel), etc.

In some embodiments, other data of the patient is also input into the trained machine-learning model, such as data related to one or more interventional devices used for the treatment (e.g., type, size, escalation in size during procedure, flexibility, force applied to device when entering occlusion, location of device entry of occlusion with respect to vessel wall) and personal data of patient (e.g., age, blood pressure, weight, heart health, disease history, smoking history, and family health history). The current treatment plan used for the treatment procedure is also input into the machine-learning model.

The method, in block 576, predicts by the machine-learning model whether to adjust the treatment plan. To do so, using the treatment progress characteristics, the machine-learning model may determine the probability of a successful outcome of the treatment procedure using the current treatment plan. Improved information may be available of the treatment progress characteristics as inter-operative data becomes available during the treatment procedure (including from results of actions performed during the procedure, such as deforming of a guidewire inserted during procedure). Such improved information may cause the determination of the probability of a successful outcome of the treatment procedure to change. The machine-learning model may compare the current determined probability of a successful outcome to an earlier determination of the probability of a successful outcome, such as determined earlier during the treatment procedure and stored in memory or from pre-operative data. If the probability of a successful outcome has changed (decreased), the machine-learning model may predict that an adjustment is needed to the treatment plan. In some embodiments, the machine-learning model may also, using the treatment progress characteristics, determine the probability of a successful outcome using different options for the treatment selections in the treatment plan. If a different option results in an improved probability of a successful outcome, the machine-learning model may predict that an adjustment is needed to the treatment plan.

If the method predicts an adjustment is needed, the method, in block 578, predicts by the machine-learning model the specific adjustments to the treatment selections in the treatment plan. For example, using the treatment progress characteristics, the machine-learning model may determine the probability of a successful outcome with the current selected option for each treatment selection and the probabilities of a successful outcome with the alternative options for each treatment selection. If an alternative option for a treatment selection has a higher probability of successful outcome than the current selected option, the machine-learning model may predict adjustment of the treatment selection to the alternative option. For example, intra-operative images during the treatment procedure may capture the deforming of a guidewire making contact with the occlusion and the machine-learning model may determine that the manner in which the guidewire deformed is indicative of a stiff proximal cap. From such determination, the machine-leaning model may determine that the probability of a successful outcome of an antegrade approach (as currently in treatment plan) is lower than originally determined from pre-operative image data and that the probability of successful outcome of an alternative retrograde approach now has a higher probability of successful outcome. Thus, the machine-learning model predicts the specific adjustment to the approach direction treatment selection to change from antegrade to retrograde.

Fig. 6A illustrates a schematic diagram of training of a machine-learning model to predict adjustment of a treatment plan, in accordance with some embodiments of the present disclosure.

Fig. 6A includes a machine-learning model 670. In some embodiments, the machine-learning model 670 may a neural network 170 that may include one or more architectures such as such as an artificial neural network (ANN), a convolutional neural network (CNN), a recurrent neural network (RNN), variational autoencoder (VAE), a transformer, a U-net model, and so forth. In one example embodiment, the machine-learning model 670 may be a deep learning model, such as described in "Deep Learning-based Prediction of Percutaneous Recanalization in Chronic Total Occlusion Using Coronary CT Angiography", Zhou et al., Radiology, Vol 309, No. 2, November 2023, which is incorporated herein by reference. The training of the machine-learning model 670 comprises inputting a training dataset into the machine-learning model 670 and algorithms of the machine-learning model executing using the input to generate a trained treatment prediction model 690.

As shown in Fig. 6A, the training dataset may include historical images 610 related to past treatments, such as for treating an occlusion (e.g., CTO). For example, the training data may include historical images 610 from a relatively large patient population (e.g., greater than 100) of patients who have undergone similar treatment procedures (e.g., PCIs) to treat occlusions. The historical images may include pre-operative images of the past treated patients and/or intra-operative images of past treatment procedures of the past treated patients. The historical images 610 may include image features of the past patients' anatomies, such as treatment progress characteristics related to occlusions within the past patients, for example morphologies of proximal caps and/or distal caps of the occlusions, quality of vessels distal to the occlusions, and/or collateral vessels of the occlusions. The historical images 610 may capture the results of actions treating the occlusions, such as the bending and/or deforming of a guidewire inserted into the occlusions during the past treatment procedures.

The training dataset may also include historical treatment plans 618 corresponding to the historical images 610 and outcomes of the past treatment procedures corresponding to the historical images 610. The training data may also include historical treatment device data 615 corresponding the historical images 610, such as types of treatment devices used in the past treatment procedures, properties of the historical treatment devices (e.g., size, flexibility), reactions to the historical treatment devices (e.g., forced applied by devices when contacting occlusions) measured by sensors positioned on or near the historical treatment devices, etc. The training data may also include data or metrics 616 of the past patients (e.g., age, weight, blood pressure, heart health, smoking history, family health history, past treatment procedure results, etc.) that may affect efficacy and/or success rate of the previous treatment procedures.

In embodiments, the machine-learning model may be trained in a supervised, unsupervised, semi-supervised, or reinforced manner to implement the operations to predict the adjustment of a treatment plan. In some supervised and semi-supervised embodiments, the historical images 610 include annotation of image features in the historical images related to the occlusions. The annotated image features may include properties related to the treatment progress characteristics of the occlusions, such as properties of the proximal cap (e.g., location, shape, stiffness), the distal cap (e.g., location, shape, stiffness), the vessel distal to the occlusion (e.g., location, size, damage), the collateral vessels (location, tortuosity, angle to vessel), and interventional device (type, size, location with respect to occlusion, bending, deformation) used for treating the occlusions.

Such image features may be annotated manually using any annotation tools, such as bounding box, for example, or automatically using any known image processing or computer vision approaches. In some embodiments, such image features may be automatically extracted from the historical images 610 for the supervised or semi-supervised training by the machine-learning model. The historical images 610 may be input to a known image processing or computer vision application to extract the image features. The application may also be used prior to input to the model to filter the images to remove particular images not of suitable image quality or not having suitable visualization of image features of the occlusion (such as unclear visualization of the proximal cap), or the application may enhance visualization of the image features of the occlusion in particular historical images, such as stacking the particular historical images (as provided in Philips StentBoost).

The historical treatment plans 618 of the training data may further include the treatment selections used to treat the occlusion in the corresponding historical images 610 in the past treatment procedures (e.g., an antegrade approach, retrograde approach, or dual approach treatment selection was used to treat the occlusion in the historical image) and the ground truth outcome of the past treatment procedure. The outcome may also be demonstrated as binary outcomes, such as success or failure outcome of treating the occlusion. Alternatively, or in addition, the ground truth outcomes may be demonstrated as qualitative outcomes grading the level of success of treating the occlusion. For example, the qualitative outcomes may include treatment results, such as "occlusion successfully treated," "failed to cross occlusion in antegrade direction due to proximal cap stiffness," "failed to cross occlusion in antegrade direction due to blunt shape of proximal cap," "failed to cross occlusion due to length of occlusion > 20mm," "failed to cross occlusion due to calcification of the occlusion," "failed to cross occlusion in retrograde direction tortuosity of collateral vessels," etc. In some embodiments, the annotations may include a score indicating the level of difficulty that the image features cause to the success of the treatment procedure, such as "blunt shape of proximal cap, score: 1" to indicate increased antegrade approach difficulty due to blunt shape. An example of score annotations is shown in Fig. 7B. Of course, other annotations indicating the levels of success and results of the occlusion treatment procedure may be included without departing from the scope of the present teachings.

In some supervised and semi-supervised embodiments, the machine-learning model 670 is trained using the annotated historical data and ground truth data to predict the probabilities of a successful outcome with regard to the treatment procedure and/or individual treatment selections in the treatment procedure, and to determine levels of confidence with regard to the predicted probabilities. In some embodiments, the training may determine and analyze relationships or correlations between the features related to the occlusion in the historical images 610 with respect to the ground truth annotated data to train the machine-learning model to predict the probabilities of successful outcome of a particular treatment selection option (e.g., antegrade, retrograde, or dual approach to occlusion). For example, the training may correlate, in the historical images, the bending of the guidewire in contact with the proximal cap and the tortuosity of the septal collateral vessel with respect to the ground truth annotated outcomes of "failed to cross occlusion in antegrade direction due to proximal cap stiffness" and "succeeded to cross occlusion in retrograde direction using septal collateral vessel", to train the machine-learning model 670 (as the trained treatment prediction model 690) to predict probability of a successful outcome of a retrograde approach.

The training may include training the machine-learning model to predict the probability of successful outcome of different treatment selections (such as probability of successful outcome of antegrade approach and of retrograde approach) and selecting the treatment selection with the highest predicted probability of success. The training may also include training the machine-learning model to predict probability of successful outcome of all treatment selections options to be insufficiently low (e.g., less than 50%) and predict adjustment to terminate the treatment procedure. In some embodiments, the predicted probability of success may be determined as a treatment difficulty score based on scores assigned to the image features. The treatment difficulty score may be determined based on score annotations of the image features in the historical images (such as shown in Fig. 7B), analysis of the image features using predetermined rules (e.g., scoring criteria in Fig. 7A), analysis and correlation of the same image feature in a large number of images and corresponding treatment outcome, etc. In some embodiments, the predetermined criteria includes anatomical rules that are also input into the machine-learning model as part of the training dataset.

When the machine-learning model is implemented as a neural network, the neural network may include several fully connected or convolutional layers with pooling, normalization, and non-linear layers between them, for example. When optional information is included (e.g., patients' personal data), this information may be incorporated by appending it to flattened feature layers before applying linear transformations followed by non-linearities, such as sigmoid functions and rectified linear units (ReLU), for example. The neural network may directly produce an $n \times 1$ vector output, where n is the number of values regressed by the neural network, such as probabilities of the successful outcome of each treatment selection option for each type of treatment selection and a total probability of a successful outcome for the treatment procedure using the highest probability treatment selection options.

Errors are computed by comparing the output values produced by the neural network with ground truth values using a loss function, such as L1 or L2 loss, Huber loss, or log cosh loss, for example. The loss functions may be used to perform stochastic gradient descent to optimize network weights. This is typically done by computing derivatives of the loss function with respect to the model parameters using the chain rule. The derivatives inform the network how the model parameters must be adjusted through each training loop in order to minimize the loss function. The adjustments to model parameters are made starting from the last layer and working backwards in the network towards the first layer, which is known as backpropagation.

In other words, the training of a neural network generally involves inputting the training dataset (e.g., annotated historical images 610 together with ground truth historical treatment plans and outcome data 618) into the machine-learning model, and iteratively adjusting the model's parameters until the trained model provides an accurate output (e.g., accurately prediction of whether treatment plan needs adjustment and specific treatment selections in plan to adjust). In some embodiments, training is performed using a Graphics Processing Unit (GPU) or a dedicated neural processor such as a Neural Processing Unit (NPU) or a Tensor Processing Unit (TPU). Training may employ a centralized approach wherein cloud-based or mainframe-based neural processors are used to train the neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth.

The process of training the machine-learning model 670 (e.g. neural network) described above includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. During training, the value of a loss function is typically minimized, and training is terminated when the value of the loss function satisfies a stopping criterion. In some embodiments, training is terminated when the value of the loss function satisfies one or more of multiple criteria.

In an example, the loss function may be a minimization with respect to properties of the treatment progress characteristics of the occlusion according to Equation (1) to predict a treatment plan adjustment:

$$argmin_{x_1, x_2, x_3, x_4} \Sigma [H(\cdot) - f(x_1, x_2, x_3, x_4, \cdot)]^2 \qquad (1)$$

where $H$ is a treatment plan and $f$ is a function describing the resulting treatment plan adjustment based on the treatment progress characterists $x_1$, $x_2$, $x_3$, $x_4$, where $x_1$ is a vector of properties of the occlusion proximal cap morphology, $x_2$ is a vector of properties of the occlusion length, course, and composition, $x_3$ is a vector of properties of the vessel distal to the occlusion, course, and $x_4$ is a vector of properties of the collateral vessels. In other embodiments, other vectors (xi...n may be included in the function, such as devices characteristics, patients characteristics, etc.)

Various methods are known for solving the loss minimization problem such as methods, and so forth. The value of the loss function may be computed using functions such as the negative log-likelihood loss, the mean squared error, or the Huber loss, or the cross entropy loss. Various algorithms have been developed to implement these methods and their variants including but not limited to, Stochastic Gradient Descent (SGD), batch gradient descent, mini-batch gradient descent, Gauss-Newton, Quasi-Newton, Levenberg Marquardt, Momentum, Adam, Nadam, Adagrad, Adadelta, RMSProp, and Adamax optimizers. These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases.

The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

The machine-learning model 670 may consist of a single neural network that estimates all outputs together. Alternatively, the machine-learning model may consist of two cascaded neural networks, where a first neural network is used to predict whether an adjustment to the treatment plan is needed and a second neural network is used to predict the specific treatment selection to be adjusted in the treatment plan. The two networks may share weights and/or may be trained together via combined loss functions and a single backpropagation loop.

In some unsupervised embodiments, the training of the machine-learning model includes reconstructing the input data in order to learn a latent representation or embedding of input training data. This training is unsupervised because ground truth annotations are not required. Training for a reconstruction task may be performed using an encoder-decoder architecture, such as an auto-encoder or a variation auto-encoder, for example. The encoder is configured to compress the input data to a reduced dimensional latent embedding. The latent embedding is then used to reconstruct the input using the decoder. The machine-learning model is trained to reconstruct the input from the latent embedding using a loss function to provide a reconstruction output, where the loss function compares the reconstructed output with the training data input using, for example, L1 or L2 loss. The comparisons are then used to perform gradient descent and to update weights, as in the previous embodiment. In other embodiments, unsupervised learning may be used in which the training module 438 executes another algorithm (e.g., k-means clustering, hierarchical clustering, anomaly detection, apriori, etc.) that trains the machine-learning model from the historical data without requiring annotation of the image features.

The machine-learning model 670 may be trained to estimate the levels of confidence (or uncertainties) based on errors produced by the machine-learning model's estimate of the probabilities of successful outcome using various historical images of the treatment progress characteristics of the occlusion and the corresponding ground truths. Alternatively, the machine-learning model may be trained to estimate the levels of confidence based on errors produced by the entire estimated $n \times 1$ vectors. In addition, the neural network, in particular, may have dropout layers, which ignore the output of some number of nodes at random, producing slightly different results for the same input. The variance in the results generated from inputting the same input into the trained neural network multiple times may inform confidence.

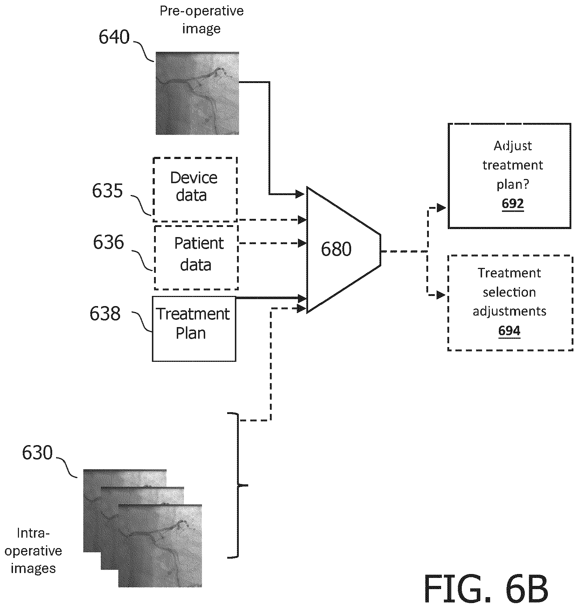

Fig. 6B illustrates a schematic diagram of an example of the trained machine-learning model 680 of Fig. 6A for predicting adjustment of a treatment plan, in accordance with some embodiments of the present disclosure. In some embodiments, the trained machine-learning model 680 is the trained machine-learning model 690 resulting from a training method described according to Fig. 6A.

The trained machine-learning model 680 is configured to obtain, as input, intra-operative images 630 of a treatment procedure performed on a patient according to a treatment plan 638. The input intra-operative images 630 may include image features capturing the treatment progress characteristics of an occlusion (e.g., CTO), including one or more of (1) proximal cap morphology of an occlusion, (2) occlusion length, course, and composition, (3) quality of the vessel distal to the occlusion, and (4) collateral circulation. These image features may more specifically include properties related to the treatment progress characteristics of patients' occlusions, such as properties of the proximal cap of an occlusion (e.g., location, shape, stiffness bending and/or deformation of device in contact with proximal cap), the distal cap of the occlusion (e.g., location, shape, stiffness), the vessel distal to the occlusion (e.g., location, size, damage), and the collateral vessels (location, tortuosity, angle to vessel). In some embodiments, the image features capture results from actions performed during the treatment procedure in relation to the treatment progress characteristics, such as the bending and/or deforming of a guidewire in contact with the proximal cap during the treatment procedure, which provides insight of the morphology (e.g., stiffness) of the proximal cap.

The trained machine-learning model 680 may also be configured to obtain, as input, at least one pre-operative image 640 of the patient. The input at least one pre-operative image 640 may also include one or more of the image features (e.g., treatment progress characteristics) described with respect to the intra-operative image 630. The trained machine-learning model 680 is also configured to obtain, as input, intra-operative device 635 data related to the treatment procedure. The intra-operative treatment device 635 may include data related to the one or more interventional devices used for the treatment, (type, size, escalation in size during procedure, flexibility) and behavior and location of the device with respect to occlusion (e.g., force applied to device when entering occlusion, location of device entry of occlusion with respect to vessel wall). The trained machine-learning model 680 may also be configured to obtain, as input, patient data 636 of the patient (e.g., age, blood pressure, weight, heart health, disease history, smoking history, and family health history). The trained machine-learning model 680 is also configured to obtain, as input, the treatment plan 638 (which may be generated from the at least one pre-operative image 640) used from the treatment procedure.

The trained machine-learning model 680 applies the input to the functions of the trained machine-learning model 680, such as the loss functions described with respect to Fig. 6A, to predict whether an adjustment 692 is needed to the input treatment plan 638. In some embodiments, the functions of the trained machine-learning model 680 predict whether such adjustment to the plan 692 is needed to the treatment plan 638 by predicting the probability of a successful outcome of the treatment procedure using the treatment plan based on analysis of the intra-operative images 630 of the progression of the treatment procedure.

For example, the trained machine-learning model 680 may determine that based on analysis of the current intra-operative images 630 captured of the treatment procedure using the treatment plan 638 has a lower probability of a successful outcome (e.g., 40%) than earlier determined (e.g., 70%), which indicates that an adjustment 692 is need to the treatment plan. In some embodiments, the earlier determined probability may be determined from intra-operative data capture earlier during the treatment procedure and stored in memory or determined from pre-operative data prior to the treatment procedure. In some embodiments, the trained machine-learning model 680 may analyze the current intra-operative images 630 together with one or more of the at least one pre-operative image 640, the device data 635, and the patient data 636 to predict the probability of a successful outcome of the treatment procedure.

To predict the need to adjust the treatment plan, in some embodiments, the machine-learning model 680 may determine the probability of a successful outcome with the option selected for each treatment selection in the treatment plan 638 and also with alternative options for each treatment selection in the treatment plan 638. As improved information may be available from the intra-operative data (e.g., intra-operative images 630) of the treatment procedure, the assessment of probability of success of the treatment options for a treatment selection may change. For example, based on the intra-operative input data, the trained machine-learning model 680, may determine the probability of a successful outcome with the option of an antegrade (currently selected in the treatment plan 638) and the probabilities of a successful outcome with the alternative options of a retrograde and duel antegrade-retrograde for the treatment selection of occlusion approach direction. From the improved intra-operative data, the machine-learning model 680 may now determine that the probability of a successful outcome with an alternative option (e.g., retrograde approach) for the treatment selection is higher than the current selection (e.g., antegrade approach) and output the prediction that an adjustment is needed to the treatment plan 638. The machine-learning model 680 may instead determine that no alternative option is available for the treatment selections that would increase the probability of a successful outcome of the treatment procedure. The machine-learning model 680 may then output the prediction that no adjustment is needed, or if the probability of a successful outcome is below a predetermined threshold (e.g., below 50%), machine-learning model 680 may output the prediction to adjust the treatment plan to terminate the treatment procedure.

In some embodiments, if from the improved intra-operative data, the machine-learning model 680 determines that the probability of a successful outcome with an alternative option (e.g., retrograde approach) for a treatment selection is higher than the current selection (e.g., antegrade approach), the machine-learning model 680 may output the prediction to adjust 694 that treatment selection (e.g., adjusting from antegrade to retrograde).

In some embodiments, the prediction of the machine-learning model 680 may be output (e.g., via display 424 of Fig. 4A) as a recommendation to the user, and the user decides whether or not to adjust the treatment plan 638 according to the recommendation. In other embodiments, the prediction of the machine-learning model 680 may automatically be used (e.g., via by controller 410 of Fig. 4A) to update the treatment plan. In some embodiments, the machine-learning model 680 may also determine levels of confidence associated with its prediction of the probability of successful outcome of the treatment and treatment selections. The levels of confidence may factor into the prediction of whether an adjust 692 is recommended for the treatment plan and/or the recommendation of the treatment selection option to adjust 694. In some embodiments, the level of confidence may be output along with the recommendation to the user.

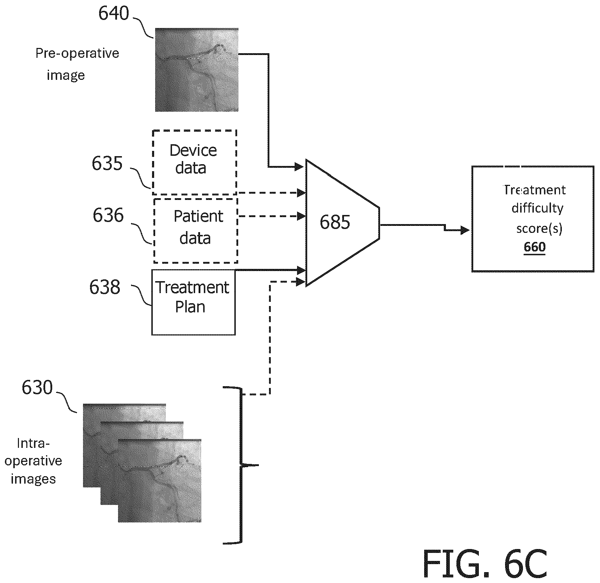

Fig. 6C illustrates a schematic diagram of a trained machine-learning model for predicting treatment difficulty scores 660 for adjusting a treatment plan, in accordance with some embodiments of the present disclosure. In some embodiments, the trained machine-learning model 685 is the trained machine-learning model 690 resulting from a training method described according to Fig. 6A.

The trained machine-learning model 680 is configured to obtain, as input, intra-operative images 630 of a treatment procedure performed on a patient according to the treatment plan 638 (as described with respect to Fig. 6B). The trained machine-learning model 680 may also be configured to obtain at least one pre-operative image 640; intra-operative device data 635; and/or patient data (as described with respect to Fig. 6B).

The trained machine-learning model 680 applies the input to the functions of the trained machine-learning model 680, such as the loss functions described with respect to Fig. 6A, to predict whether an adjustment 692 is needed to the input treatment plan 638. In some embodiments, the functions predicts the probability of a successful outcome of the treatment procedure as one or more treatment difficulty scores. In these embodiments, the machine-learning model may be trained with historical annotated images 610, such as in Fig. 7B, in which scores are assigned to properties of the treatment progress characteristics in the historical annotated images or anatomical rules that define scoring of the properties of the treatment progress characteristics, such as in Fig. 7A. In some embodiments, the anatomical rules 620 are input into the trained machine-learning model for scoring of the properties of the treatment progress characteristics by the machine-learning model.

For example, the trained machine-learning model 680 may determine that based on analysis of the current intra-operative images 630, the treatment plan 638 has a higher difficulty score 660 (e.g., 4) than earlier determined (e.g., 2), which indicates that an adjustment is need to the treatment plan. Such higher difficulty score may indicate a lower probability of success outcome of the treatment procedure. In some embodiments, the earlier determined difficulty score may be determined from intra-operative data capture earlier during the treatment procedure and stored in memory or determined from pre-operative data prior to the treatment procedure. In some embodiments, the treatment difficulty score 660 for the treatment plan is determined by combining the treatment difficulty scores assigned by the machine-learning model to each treatment selection in the treatment plan. In some embodiments, the trained machine-learning model 680 may analyze the current intra-operative images 630 together with one or more of the at least one pre-operative image 640, the device data 635, and the patient data 636 to predict the difficulty score of the treatment procedure.

To predict the need to adjust the treatment plan, in some embodiments, the machine-learning model 680 may determine the difficulty score for the option selected for each treatment selection in the treatment plan 638 and also for alternative options for each treatment selection in the treatment plan 638. As improved information may be available from the intra-operative data (e.g., intra-operative images 630) of the treatment procedure, the assigned treatment difficulty score for a treatment selection may change. For example, based on the intra-operative input data, the trained machine-learning model 680, may determine the treatment difficulty score for the option of antegrade (currently selected in the treatment plan 638) and for the alternative options of a retrograde and duel antegrade-retrograde for the treatment selection of occlusion approach direction. From the improved intra-operative data, the machine-learning model 680 may now determine that the treatment score for an alternative option (e.g., retrograde) for the treatment selection is lower than the current selection (e.g., antegrade) and output the prediction that an adjustment is needed to the treatment plan 638. The machine-learning model 680 may instead determine that no alternative option is available for the treatment selections that would improve the treatment difficulty score of the treatment procedure. The machine-learning model 680 may then output the prediction that no adjustment is needed, or if the combined treatment difficulty score for all the treatment selections is above a predetermined difficulty threshold (e.g., above 3), machine-learning model 680 may output the prediction to adjust the treatment plan to terminate the treatment procedure.

In some embodiments, if from the improved intra-operative data, the machine-learning model 680 determines that the treatment difficulty score for an alternative option (e.g., retrograde) for a treatment selection is lower than the difficulty score for a current selection (e.g., antegrade), the machine-learning model 680 may output the recommendation to adjust that treatment selection (e.g., adjusting from antegrade to retrograde).

In some embodiments, the prediction of the machine-learning model 680 may be output (e.g., via display 424 of Fig. 4A) as a recommendation to the user, and the user decides whether or not to adjust the treatment plan 638 according to the recommendation. In other embodiments, the prediction of the machine-learning model 680 may automatically be used (e.g., via by controller 410 of Fig. 4A) to update the treatment plan. In some embodiments, the machine-learning model 680 may also determine the levels of confidence associated with its prediction of the treatment difficulty scores for the treatment and treatment selections and output the levels of confidence along with the recommendations to the user.

Fig. 7A illustrates example scores for characteristics related to an occlusion to select a treatment for the occlusion, in accordance with some embodiments of the present disclosure.

The scoring of Fig. 7A includes assigning a score to the occlusion characteristics of proximal cap shape ("0" for tapered or "1" for blunt), calcification in the occlusion ("0" for absence or "1" for presence), bending of a segment of the occlusion ("0" for absence or "1" for presence), occlusion length ("0" for <= 20 mm or "1" > 20 mm), and re-try attempt to enter the occlusion ("0" for No or "1" for Yes). The score is assigned based whether the characteristic increases the difficulty of the treatment procedure. The scores of each characteristic is total and the total score compared to threshold levels of difficult ("0" for Easy; "1" for Intermediate; "2" for Difficult; >= 3 for Very Difficult).

In some example embodiments, the rules used to determine adjusts to the treatment plan may be based at least in part on the scoring strategy show in Fig. 7A, such as the rules described as being used by the scoring engine 437 of Fig. 4B, scoring method of Fig. 5B, or machine-learning model of Fig. 6C. In an example embodiments, the scores may be used as part of a grading model, as described in "Predicting Successful Guidewire Crossing Through Chronic Total Occlusion of Native Coronary Lesions Within 30 Minutes", JCC: Cardiovascular Intervention, The American College of Cardiology Foundation, Vol. 4, No. 2, ISSN 1936-8798, 2011, which is incorporated herein by reference.

Fig. 7B illustrates an example scored image for selecting treatment for an occlusion, in accordance with some embodiments of the present disclosure.

The image of Fig. 7B includes a proximal cap with a tapered (concave) shape that is annotated with the score of "0" due to a tapered shape cap being less difficult to enter than a blunt shaped proximal cap. The image also includes an occlusion length that is annotated with the score of "0" due to the length being less than 22mm (which is less difficult to treat than an over 22mm length). The image further includes a calcification score that is annotated with the score of "1" due to the large amount of calcification causing difficulty for crossing the occlusion. The image also includes a distal vessel that is annotated with the score of "0" due to lack of disease causing less difficulty to traverse. The image also includes collateral vessels that are annotated with the score of "0" due to lack of tortuosity causing less difficulty to traverse. In some embodiments, these scored features of the image may be used to determine adjustment of a treatment plan, such as by the scoring engine 437 of Fig. 4B, scoring method of Fig. 5B, or machine-learning model of Fig. 6C.

Although the present specification describes components and functions that may be implemented in particular embodiments with reference to particular standards and protocols, the disclosure is not limited to such standards and protocols. Such standards are periodically superseded by more efficient equivalents having essentially the same functions. Accordingly, replacement standards and protocols having the same or similar functions are considered equivalents thereof.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents, and shall not be restricted or limited by the foregoing detailed description.

## Claims

1. A system for determining a treatment plan for a medical procedure, the system comprising:
   a processor in communication with memory, the processor configured to:

   generate the treatment plan for the medical procedure,
   obtain one or more images acquired during performance of the medical procedure according to the treatment plan,
   record progression of the medical procedure based on the one or more images,

extract at least one treatment progress characteristic from the one or more images, and
determine whether to adjust the treatment plan based on the at least one treatment. progress characteristic and the progression of the medical procedure.

2.  The system of claim 1, wherein, if determined to adjust the treatment plan, the processor is further configured to determine an adjustment to a treatment selection in the treatment plan based on the at least one treatment progress characteristic and the progress of the medical procedure.

3.  The system of claim 1, wherein:

    the treatment plan is for treatment of an occlusion of a vessel, and
    the at least one treatment progress characteristic includes at least one of: (i) proximal cap morphology of an occlusion, (ii) occlusion length, course, and composition, (iii) quality of a vessel distal to the occlusion, and (iv) collateral circulation.

4.  The system of claim 1, wherein the processor is further configured to determine whether to adjust the treatment plan based on data acquired by a sensor disposed on a treatment device used to perform the medical procedure according to the treatment plan.

5.  The system of claim 1, wherein the processor is further configured to score one or more properties of the at least one treatment progress characteristic to determine at least one of (i) whether to adjust the treatment plan and (ii) a preferred treatment selection to use to adjust the treatment plan.

6.  The system of claim 1, wherein the processor is further configured to determine one or more properties of the at least one treatment progress characteristic based on a result of an action performed during the medical procedure, including at least one of: bending and deforming of a treatment device inserted into a vessel during progression of the medical procedure, position of the treatment device with respect to a wall of the vessel, force asserted on the treatment device in response to contact with the vessel or an occlusion in the vessel.

7.  The system of claim 1, wherein the processor is configured to input the treatment plan and one or more properties of the at least one treatment progress characteristic into a machine-learning model trained to predict at least one of (i) whether to adjust the treatment plan and (ii) a preferred treatment selection to use to adjust the treatment plan.

8.  A method of determining a treatment plan for a medical procedure, the method comprising:

    generating the treatment plan for the medical procedure;
    obtaining one or more images acquired during performance of the medical procedure according to the treatment plan;
    recording progression of the medical procedure based on the one or more images;
    extracting at least one treatment progress characteristic from the one or more images; and
    determining whether to adjust the treatment plan based on the at least one treatment progress characteristic and the progression of the medical procedure.

9.  The method of claim 8, wherein, if determined to adjust the treatment plan, the method further comprising determining an adjustment to a treatment selection in the treatment plan based on the at least one treatment progress characteristic and the progress of the medical procedure.

10. The method of claim 8, wherein:

    the treatment plan is for treatment of an occlusion of a vessel, and
    the at least one treatment progress characteristic includes at least one of: (i) proximal cap morphology of an occlusion, (ii) occlusion length, course, and composition, (iii) quality of a vessel distal to the occlusion, and (iv) collateral circulation.

11. The method of claim 8, further comprising:
    inputting the treatment plan and one or more properties of the at least one treatment progress characteristic into a machine-learning model trained from images of past procedures to predict at least one of (i) whether to adjust the treatment plan and (ii) a preferred treatment selection to use to adjust the treatment plan.

12. A non-transitory computer-readable storage medium having stored a computer program comprising instructions for determining a treatment plan for a medical procedure, the instruction, when executed by a processor, further cause the processor to:

generate the treatment plan for the medical procedure;
obtain one or more images acquired during performance of the medical procedure according to the treatment plan;
record progression of the medical procedure based on the one or more images;
extract at least one treatment progress characteristic from the one or more images; and
determine whether to adjust the treatment plan based on the at least one treatment progress characteristic and the progression of the medical procedure.

13. The non-transitory computer-readable storage medium of claim 12, wherein, if determined to adjust the treatment plan, the instructions, when executed by the processor, further cause the processor to determine an adjustment to a treatment selection in the treatment plan based on the at least one treatment progress characteristic and the progress of the medical procedure.

14. The non-transitory computer-readable storage medium of claim 12, wherein:

the treatment plan is for treatment of an occlusion of a vessel, and
the at least one treatment progress characteristic includes at least one of: (i) proximal cap morphology of an occlusion, (ii) occlusion length, course, and composition, (iii) quality of a vessel distal to the occlusion, and (iv) collateral circulation.

15. The non-transitory computer-readable storage medium of claim 12, wherein the instructions, when executed by a processor, further cause the processor to:
input the treatment plan and one or more properties of the at least one treatment progress characteristic into a machine-learning model trained from images of past procedures to predict at least one of (i) whether to adjust the treatment plan and (ii) a preferred treatment selection to use to adjust the treatment plan.

FIG. 1A

EP 4 685 809 A1

FIG. 1B

EP 4 685 809 A1

FIG. 2

**Example Treatment Selections**

**Example Options**

Approach Direction
(310)

Antegrade
(311)

Retrograde
(312)

Dual
(313)

Contract Injection
(320)

Yes
(321)

No
(322)

Dual
(323)

Crossing Technique
(330)

True lumen/ wiring
(331)

Dissection & Reentry
(select technique)
(332)

Treatment Device
Selections
(340)

Guidewire type
selection &
escalation
(341)

Catheter type
selection
(342)

# FIG. 3

EP 4 685 809 A1

FIG. 4A

Treatment Plan Module **432**

Training Module

**438**

Prediction Module

**436**

Scoring Engine

**437**

Plan Generator

**433**

Plan Adjuster

**434**

# FIG. 4B

FIG. 4C

EP 4 685 809 A1

500

Start

↓

Generate a treatment plan
for a medical procedure — 510

↓

Acquire images of progression of
procedure according to the plan — 520

↓

Record progression of procedure
based on the images — 530

↓

Extract treatment progress
characteristics from the images — 540

↓

Determine whether to adjust plan
based on treatment progress
characteristics — 550

↓ Yes

Determined adjustments to treatment
selections in plan based on treatment
progress characteristics — 560

↓

End

FIG. 5A

550, 560

```
        ┌────────┐
        │  540   │
        └────────┘
            │
            ▼
┌──────────────────────────────┐
│ Identify one or more          │
│ properties of the treatment   │── 551
│ progress characteristics      │
└──────────────────────────────┘
            │
            ▼
┌──────────────────────────────┐
│ Access historical data and/or │
│ rules related to the one or   │── 552
│ more properties               │
└──────────────────────────────┘
            │
            ▼
┌──────────────────────────────┐
│ Score the one or more         │
│ properties based on           │── 553
│ historical data and/or rules  │
└──────────────────────────────┘
            │
            ▼
┌──────────────────────────────┐
│ Determine treatment           │
│ difficulty score based on the │── 554
│ one or more property scores   │
└──────────────────────────────┘
            │
            ▼
┌──────────────────────────────┐
│ Determine whether to adjust   │
│ plan based on treatment       │── 555
│ difficulty score              │
└──────────────────────────────┘
            │ Yes
            ▼
┌──────────────────────────────┐
│ Determine adjustments to      │
│ treatment selections in plan  │── 556
│ based on treatment difficulty │
│ score                         │
└──────────────────────────────┘
            │
            ▼
        ┌────────┐
        │  End   │
        └────────┘
```

# FIG. 5B

550, 560

540

Obtain trained machine-learning model — 572

Input treatment progress characteristics into model — 574

Predict, by model, whether to adjust plan — 576

Yes

Predict, by model, adjustments to treatment selections in plan — 578

End

FIG. 5C

FIG. 6A

FIG. 6B

FIG. 6C

FIG. 7A

EP 4 685 809 A1

FIG. 7B

Europäisches
Patentamt

European
Patent Office

Office européen
des brevets

**EUROPEAN SEARCH REPORT**

Application Number

EP 24 30 6255

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/093712 A1 (BUSCH ERIK [DE] ET AL) 9 April 2009 (2009-04-09)<br>* paragraph [0018] *<br>* paragraph [0025] - paragraph [0029] *<br>* paragraph [0046] *<br>* paragraph [0050] - paragraph [0052] *<br>* figures 1-4 *<br>----- | 1-15 | INV.<br>G16H20/40<br>A61B5/02<br>A61B34/10<br>A61B34/20<br>G16H30/40<br>G16H50/20<br>G16H50/30<br>G16H50/50 |
| X | WO 2024/058837 A1 (MEDTRONIC VASCULAR INC [US]) 21 March 2024 (2024-03-21)<br>* paragraph [0007] *<br>* paragraph [0071] *<br>* paragraph [0091] *<br>* paragraph [0127] *<br>* paragraph [0187] *<br>* paragraph [0213] *<br>* paragraph [0286] - paragraph [0289] *<br>* figures 1, 2 *<br>----- | 1-15 | |
| A | REMPAKOS ATHANASIOS ET AL: "Predicting Successful Chronic Total Occlusion Crossing With Primary Antegrade Wiring Using Machine Learning",<br>JACC: CARDIOVASCULAR INTERVENTIONS, ELSEVIER, AMSTERDAM, NL,<br>vol. 17, no. 14, 3 July 2024 (2024-07-03), pages 1707-1716, XP087568118,<br>ISSN: 1936-8798, DOI:<br>10.1016/J.JCIN.2024.04.043<br>[retrieved on 2024-07-03]<br>* the whole document *<br>* figures 3, 4 *<br>----- | 1-15 | TECHNICAL FIELDS SEARCHED (IPC)<br><br>G16H<br>A61B |

-/--

The present search report has been drawn up for all claims

| Place of search | Date of completion of the search | Examiner |
|---|---|---|
| Munich | 13 December 2024 | Hauber, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 30 6255

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KARATASAKIS ARIS ET AL: "Approach to CTO Intervention: Overview of Techniques", CURRENT TREATMENT OPTIONS IN CARDIOVASCULAR MEDICINE, CURRENT SCIENCE INC., PHILADELPHIA, PA, US, vol. 19, no. 1, 20 January 2017 (2017-01-20), pages 1-17, XP036140572, ISSN: 1092-8464, DOI: 10.1007/S11936-017-0501-2 [retrieved on 2017-01-20] * the whole document * * figure 1 * ----- | 1-15 | |
| A | ANANTHA-NARAYANAN MAHESH ET AL: "Contemporary Approach to Chronic Total Occlusion Interventions", CURRENT TREATMENT OPTIONS IN CARDIOVASCULAR MEDICINE, CURRENT SCIENCE INC., PHILADELPHIA, PA, US, vol. 21, no. 1, 18 January 2019 (2019-01-18), pages 1-15, XP036677930, ISSN: 1092-8464, DOI: 10.1007/S11936-019-0704-9 [retrieved on 2019-01-18] * the whole document * * Scoring systems for CTO; page 4 - page 5 * ----- | 1-15 | TECHNICAL FIELDS SEARCHED (IPC) |

The present search report has been drawn up for all claims

| Place of search | Date of completion of the search | Examiner |
|---|---|---|
| Munich | 13 December 2024 | Hauber, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 30 6255

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2024

| Patent document cited in search report | Publication date | Patent family member(s) | Publication date |
|---|---|---|---|
| US 2009093712 A1 | 09-04-2009 | DE 102008031146 A1<br>US 2009093712 A1 | 16-04-2009<br>09-04-2009 |
| WO 2024058837 A1 | 21-03-2024 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BRILAKIS**. Manual of Chronic Total Occlusion Interventions: A Step-by-Step Approach. Academic Press, Elsevier, 2022
- **BRILAKIS et al.** A Percutaneous Treatment Algorithm for Crossing Coronary Chronic Total Occlusion. JACC: Cardiovascular Interventions, The American College of Cardiology Foundation, 2012, vol. 5, 367-379
- Guiding Principles for Chronic Total Occlusion Percutaneous Coronary Intervention: A Global Expert Consensus Document. **BRILAKIS et al.** Circulation. 05 July 2019, vol. 140, 420-433
- **ZHOU et al.** Deep Learning-based Prediction of Percutaneous Recanalization in Chronic Total Occlusion Using Coronary CT Angiography. *Radiology*, 02 November 2023, vol. 309
- Predicting Successful Guidewire Crossing Through Chronic Total Occlusion of Native Coronary Lesions Within 30 Minutes. JCC: Cardiovascular Intervention. The American College of Cardiology Foundation, 2011, vol. 4